(19) Europäisches Patentamt European Patent Office Office européen des brevets

(11) **EP 2 684 150 B1**

(12) **EUROPEAN PATENT SPECIFICATION**

(45) Date of publication and mention of the grant of the patent:
**10.08.2022  Bulletin 2022/32**

(21) Application number: **12709831.7**

(22) Date of filing: **09.03.2012**

(51) International Patent Classification (IPC):
**G06F 16/33** (2019.01)   **G06Q 30/02** (2012.01)
**G16B 25/00** (2019.01)

(52) Cooperative Patent Classification (CPC):
**G06F 16/334; G06F 16/951; G06F 16/9535;**
**G06Q 30/02; G16B 25/10; G16B 40/00;**
G16B 25/00

(86) International application number:
**PCT/EP2012/054160**

(87) International publication number:
**WO 2012/123374 (20.09.2012 Gazette 2012/38)**

(54) **METHOD FOR ROBUST COMPARISON OF DATA**

VERFAHREN ZUM ROBUSTEN DATENVERGLEICH

PROCÉDÉ PERMETTANT UNE COMPARAISON ROBUSTE DE DONNÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.03.2011  EP 11157794**

(43) Date of publication of application:
**15.01.2014  Bulletin 2014/03**

(73) Proprietor: **Qlucore AB**
**223 70 Lund (SE)**

(72) Inventors:
• **FONTES, Magnus**
**224 75 Lund (SE)**
• **SONESON, Charlotte**
**22643 Lund (SE)**

(74) Representative: **Ström & Gulliksson AB**
**P.O. Box 4188**
**203 13 Malmö (SE)**

(56) References cited:
• **Giuseppe Jurman ET AL: "Algebraic Comparison of Partial Lists in Bioinformatics", arXiv:1004.1341, 8 April 2010 (2010-04-08), XP55043087, Retrieved from the Internet: URL:http://arxiv.org/pdf/1004.1341.pdf [retrieved on 2012-11-05]**
• **Stefanie Scheid ET AL: "Similarities of Ordered Gene Lists User's Guide to the Bioconductor Package OrderedList 1.3.5", Max Planck Institute for Molecular Genetics Computational Diagnostics Group @ Dept. Vingron Technical Report Nr. 2006/01, 31 December 2006 (2006-12-31), XP55043093, Retrieved from the Internet: URL:http://compdiag.molgen.mpg.de/techreports/tr_2006_01.pdf [retrieved on 2012-11-05]**

• **Ronald Fagin ET AL: "Comparing top k lists", Proceeding SODA '03 Proceedings of the fourteenth annual ACM-SIAM symposium on Discrete algorithms, 31 December 2003 (2003-12-31), pages 28-36, XP55043094, ISBN: 0898715385 Retrieved from the Internet: URL:http://delivery.acm.org/10.1145/650000 /644113/p28-fagin.pdf?ip=145.64.134.241&ac c=ACTIVE SERVICE&CFID=137165612&CFTOKEN=744228 18&__ acm__=1352124977_a3bf7e414cb3a244aa9b695 f3 1b45de7 [retrieved on 2012-11-05]**

**Description**

**Technical Field**

This invention pertains in general to the field of bioinformatics. More particularly the invention relates to a computer-implemented method for analyzing multivariate data from genetic studies.

**Background**

Many modern experimental techniques make it possible to study a large number of biological variables in a single experiment. For example, with the microarray technique researchers can monitor the activity of tens of thousands of genes simultaneously. Other measurement techniques, such as next generation sequencing, provide the possibility to study many aspects of genomics and proteomics and provide data sets with even higher number of variables. Clearly, to make sense of such a vast data set, it is necessary to be able to rank the variables in order of importance for a particular application. Having such a ranking, the researcher can select a number of top-ranked genes that are strongly linked to the studied condition and that should therefore be studied in more depth. Methods for generating variable rankings from experimental results are abundant. For example, the genes can be ranked by their relative increase (or decrease) in activity among breast cancer patients compared to healthy persons, in order to get a deeper understanding of risk factors and causes of breast cancer. Obviously, to make it possible for the researcher to draw generalizable and biologically relevant conclusions the ranking of the genes should not depend significantly on which particular cancer patients and healthy controls that are included in the study. In practice, however, it has been shown in several studies that there is a strong dependence on the selected sample (Fortunel et al, 2003; Michiels et al, 2005; Fan et al, 2006; Abraham et al, 2010). For example, Abraham et al (2010) studied data from breast cancer patients classified as high risk patients or low risk patients based on the time to occurrence of distant metastasis (data set from Ivshina et al, 2006). They concluded that the ranked list of genes that were differentially expressed between high and low risk patients was extremely sensitive to the choice of patients. A single gene could easily be ranked anywhere between position 1 and 1,500 (among 22,215 genes) if different subsets of the patients were used as the basis for the gene ranking. Similarly, Soneson and Fontes (2012) noted, using a data set comparing lung cancer patients with good and poor outcome, respectively (Bhattacharjee et al, 2001) as well as a data set comparing breast cancer patients with different mutation status (Hedenfalk et al, 2001) that the gene list obtained with a variety of different ranking methods depended considerably on the selection of included patients. Clearly, this ambiguity makes correct determination of the top-ranked genes from a single sample almost impossible, and biological interpretation of the results extremely difficult. It has been suggested that part of the reason for the apparent instability of gene rankings is that there are many genes that have similar functions in the cell, and that only one gene from a specific pathway needs to be altered in any given patient. As a consequence, the set of top-ranked genes will depend on the constitution of the particular sample.

The goal of a biological study is almost never to just rank the genes in order of importance, but rather to compare the results to those obtained from other studies, or to databases containing current knowledge. Apparently, to obtain reliable results from such comparisons it is important to have a stable and reliable ranking of the genes from the experiment. Furthermore, to be able to make such comparisons, it is necessary to have a unified way of representing the results from different sources. For example, while the results of a microarray experiment are generally represented by the ranked list of genes, the contents of a database can be represented e.g. as small, unordered collections of genes, where the genes in each collection share similar functions in the cell. By unifying the representation, we can compare any two gene lists, whether ranked or unranked and independently of the number of included genes, within the same framework. Document "Algebraic Comparison of Partial Lists in Bioinformatics" by Jurman et al, 08-04-2010, https://arxiv.org/pdf/1004.1341.pdf defines a method for identifying a set having a relationship between a plurality of bioinformatics data samples of measurement variables.

Comparing the results from a microarray experiments to those from other studies, or to databases, helps the researcher to interpret the results in a biological context. Of course, stability and reproducibility are as important as the gene ranking. By studying the biological meaning, i.e. the indication with regards to a certain biological state, of the results instead of only the actual ranking, the results are slightly more stable and the dependence on the selection of patients is somewhat reduced. However, using current methods the stabilization, the effect is still not sufficient to allow biologically relevant conclusions to be drawn.

There is thus a need for an improved method for analyzing multivariate data from genetic studies and particularly for a method enabling a determination of relationship between samples, which relationship is indicative of a biological state, and which enables improved analysis of the biological state.

**Summary of the Invention**

An objective of the present invention is to provide relatively robust comparison of data, such as gene expression data, by providing a computer-implemented method and a computer-readable medium, that analyze data according to the appended patent claims.

A general solution according to embodiments of the invention is to use information extracted from the experimental data to obtain a more stable list, which is represented in a form allowing straightforward comparisons to other lists of predetermined data, both in ordered and unordered form.

According to the invention, a computer-implemented method according to claim 1 is defined.

In the invention, the third step of expanding comprises the steps of computing a diagonal position matrix $A_\ell$, by defining diagonal elements as:

$$(A_i)_{ii} = u(r_i)$$

where $r_i$ is the ranking statistic of variable i used in sorting the data into an ordered list $\ell$, and $u : \mathbb{R} \to \mathbb{R}$ is a monotone function; computing a diagonal global weight matrix $W_\ell$ by weighting the data; and computing an expanded experimental list vector $l_\ell$ according to a function:

$$f(A_\ell, V_\ell, W_\ell) = l_\ell.$$

The computing of an expanded experimental list vector $l_\ell$ is done according to the formula:

$$l_\ell = ((l_\ell)_1, \dots, (l_\ell)_M)$$

by letting $(l_\ell)_i = h((G_\ell)_i)$, where $G_\ell = A_\ell V_\ell W_\ell$; and $(G_\ell)_i$ is the $i$:th column of $G_\ell$ and $h : \mathbb{R}^M \to \mathbb{R}$.

The subsets of variables are pairs of variables.

The pairs of variables may be pairs of variables with an exchangeability score exceeding a predefined threshold value.

The ranking method is signal-to-noise ratio (SNR), fold change of average expression value, t-test, ANOVA, and non-parametric tests.

The second step of computing an exchangeability score of each pair of variables is total exchangeability variation score, PS, mean exchangeability score, ES(mean), maximal exchangeability score, ES(max), one-sided mean exchangeability score, oES(mean), one-sided maximal exchangeability score, oES(max), or normalized variants thereof.

Computing the global weight matrix is according to the formula:

$$(W_\ell)_{ii} = \log\left(\frac{N+1}{N_i+1}\right)$$

where $N$ is the number of lists used as reference data and $N_i$ is the number of reference lists comprising $g_i$.

where $h$ is $h_1(x) = \sum_{i=1}^{M} x_i$; $h_2(x) = min_{1 \le i \le M} |x_i|$; or $h_3(x) = ||x||$ for some norm $\| \bullet \|$ on $\mathbb{R}^M$.

The fourth step of comparing the expanded experimental list vector $l_\ell$, called $l_{\ell_1}$, with the other list vector comprising a plurality of measurement variables g, called $l_{\ell_2}$, is done by computing a similarity coefficient $s(\ell_1, \ell_2)$ according to the formula

$$s(\ell_1, \ell_2) = \frac{l_{\ell_1} \cdot l_{\ell_2}}{\|l_{\ell_1}\| \|l_{\ell_2}\|};$$

and a dissimilarity coefficient d($\ell_1,\ell_2$) according to the formula

$$\mathrm{d}(\ell_1, \ell_2) = 1 - \mathrm{s}(\ell_1, \ell_2).$$

The measurement variables are of the same kind. or of different kinds.

The bioinformatics data may be any kind of data representing a plurality i of bioinformatics data samples of each of a plurality of measurement variables g. Preferably, the bioinformatics data is bioinformatics expression data, such as DNA microarray data, RNA-seq data or real-time PCR data; microRNA data, such as RNA microarray data, RNA-seq data or real-time PCR data; DNA methylation data, such as DNA methylation microarray data; or protein expression data, such as protein microarray data, antibody array data, 2-D gel data or LC-MS data. As is appreciate by a person skilled in the art, the context of the data is what makes the data carry biological information, hence bioinformatics data. This is the subject of the ranking method, which ultimately enables indication of a biological state.

According to a second aspect, a computer program product is provided, comprising computer program code means for executing the method according to claim 1 when said computer program code means are run by an electronic device having computer capabilities.

According to a third aspect, a computer readable medium is provided, having stored thereon a computer program product comprising computer program code means for executing the method of claim 1 when said computer program code means are run by an electronic device having computer capabilities.

According to a fifth aspect, an apparatus for identifying a set having a relationship between a plurality i of bioinformatics data samples of each of a plurality of measurement variables g, corresponding to claim 1.

The present invention has the advantage over the prior art that it enables relatively robust conclusions to be drawn from the data, since they provide stability with respect to re-sampling of data. A technical advantage of this is ability to determine relationship between samples, which is indicative of a biological state, and which enables improved analysis of the biological state.

## Brief Description of the Drawings

These and other aspects, features and advantages of which the invention is capable of will be apparent and elucidated from the following description of embodiments of the present invention, reference being made to the accompanying drawings, in which

Fig. 1 is a schematic overview of the method steps according to an embodiment;
Fig. 2 is a plot illustrating exchangeability according to an embodiment, for two pairs of random variables;
Fig. 3 is a result plot according to an embodiment of the invention;
Figs 4-5 are comparative result plots;
Fig. 6 is a table showing the result according to an embodiment;
Figs 7-8 are concordance plots showing the result according to an embodiment;
Fig. 9 is a schematic illustration of an embodiment;
Fig. 10 is a schematic overview of the method steps according to an embodiment;
Fig. 11 is a part of an exchangeability matrix according to an embodiment; and
Fig. 12 is a part of an expanded list vector according to an embodiment.

## Description of embodiments

Several examples of the present invention will be described in more detail below with reference to the accompanying drawings in order for those skilled in the art to be able to carry out the

The examples do not limit the invention, but the scope of the invention is only limited by the appended patent claims.

## Exchangeability of random variables

Consider a probability triple $(\Omega, \mathcal{F}, P)$ and let $X_1, \dots, X_m$ denote random variables on $\Omega$, taking values in some space $\mathbb{M}$. Given $X_1, \dots, X_m$ we define the multivariate random variable

$$X_1 \times \dots \times X_m : \Omega \to \underbrace{\mathbb{M} \times \dots \times \mathbb{M}}_{m}$$

by $X_1$ x ... x $X_m$ ($\omega$) = ($X_1(\omega),...,X_m(\omega)$). To each random variable $X_1$x...x$X_m$ there is an associated measure $Pr_{X1x...xXm}$ defined by

$Pr_{X1\times...\times Xm}$ (A) = P({$\omega \in \Omega$; $X_1 \times ... \times X_m(\omega) \in$ A}) for $\subseteq \mathbb{M} \times ... \times \mathbb{M}$.

The concept of exchangeability of random variables was made popular by de Finetti in the 1930's. Conventionally, a finite sequence ($X_1, ... , X_m$) of random variables is called exchangeable if their joint distribution is invariant under permutation of $X_1, ... , X_m$, i.e. if

$$Pr_{X_1\times...\times X_m} = Pr_{X_{\pi(1)}\times...\times X_{\pi(m)}}$$

for each $\pi \in S_m$ (the group of permutations of {1,...,m}). This means that from a statistical point of view the order of the variables in the product is completely irrelevant. From this definition, it is clear that any sequence of independent and identically distributed (i.i.d.) random variables is exchangeable, but the reverse implication is false. The definition of exchangeability given above is rather strong, and we introduce a much weaker notion of exchangeability as follows:
*Definition 1.* The finite sequence of random variables ($X_1, ... , X_m$) is weakly exchangeable if the null sets of $Pr_{X1\times...\times Xm}$ are invariant under permutations, i.e.

$$Pr_{X_{\pi(1)}\times...\times X_{\pi(m)}} \ll Pr_{X_{\tau(1)}\times...\times X_{\tau(m)}}$$

for all $\pi, \tau \in S_m$. Here, $\mu \ll v$ denotes that the positive measure $\mu$ is absolutely continuous with respect to the positive measure $v$.
It is clear that a finite sequence of random variables ($X_1, ... , X_m$) that is exchangeable is weakly exchangeable, but that the opposite implication is false in general.

**Measure of exchangeability**

In this section we will discuss some ways to quantify the degree of exchangeability for a sequence of random variables.
*Definition 2.* Given a finite sequence of random variables ($X_1, ... , X_m$), the total exchangeability variation is given by

$$P_{X_1\times...\times X_m}^{Var} := \frac{1}{|S_m|-1} \sum_{\pi\in S_m} \left| Pr_{X_{\pi(1)}\times...\times X_{\pi(m)}} - \frac{1}{|S_m|} \sum_{\tau\in S_m} Pr_{X_{\tau(1)}\times...\times X_{\tau(m)}} \right|.$$

Here, $| \mu |$ denotes the total variation of the (real-valued) measure $\mu$.

The total exchangeability variation is a positive measure and we note that $P_{X_1x...xX_m}^{Var}(\Omega) = 0$ if and only if the sequence ($X_1, ... , X_m$) is exchangeable.

We now turn to a discrete probability space ($\Omega, \mathcal{F}, P$), where $\Omega$ is a finite set,

$$\mathcal{F} = 2^\Omega$$

is the $\sigma$-algebra consisting of all events and $P: \mathcal{F} \to [0,1]$ is a probability measure.
We let $X_1, ... , X_m$ be random variables on $\Omega$ taking values in $\mathbb{M} := \{1,..., M\}$. The support of the random variable $X_1$ x ... x $X_m$ is defined by

supp $X_1 \times ... \times X_m := \{(q_1, ... , q_m) \in \mathbb{M} \times ... \times ; Pr_{X_1\times...\times X_m} (\{(q_1, ..., q_m)\}) > 0\}$

For finite sets of discrete random variables, Definition 1 implies that ($X_1, ... , X_m$) is weakly exchangeable if

$$\text{supp}(X_1 \times ... \times X_m) = \text{supp}(X_{\pi(1)} \times ... \times X_{\pi(m)})$$

for all Therefore, to quantify the degree of weak exchangeability for a set of discrete random variables we will compare the support of the joint distributions.

Let p: $(\mathbb{M} \times ... \times \mathbb{M}) \times (\mathbb{M} \times ... \times \mathbb{M}) \to \mathbb{R}$ be a metric and define the distance between two sets $A, B \subseteq (\mathbb{M} \times ... \times \mathbb{M})$ by

$$\text{dist}_\rho(A, B) := \min_{a \in A, b \in B} \rho(a, b).$$

Furthermore, define the Hausdorff distance between the two sets by

$$HD_\rho(A, B) := \max\left(\sup_{a \in A} \text{dist}_\rho(\{a\}, B), \sup_{b \in B} \text{dist}_\rho(\{b\}, A)\right).$$

*Definition 3.* Given a finite sequence of discrete random variables $(X_1, ... , X_m)$, the maximal exchangeability distance is given by

$$ED_{X_1 \times ... \times X_m}^{max} = \frac{\sum_{\pi \in S_m} \sum_{\tau \in S_m} HD_\rho\left(\text{supp } X_{\pi(1)} \times ... \times X_{\pi(m)}, \text{supp } X_{\tau(1)} \times ... \times X_{\tau(m)}\right)}{\rho(1_m, M_m)|S_m|(|S_m| - 1)}$$

and the mean exchangeability distance is given by

$$ED_{X_1 \times ... \times X_m}^{mean}$$

$$= \frac{\sum_{\pi \in S_m} \sum_{\tau \in S_m} \mathbb{E}_{X_{\pi(1)} \times ... \times X_{\pi(m)}}\left[\text{dist}_\rho\left(X_{\pi(1)} \times ... \times X_{\pi(m)}, \text{supp } X_{\tau(1)} \times ... \times X_{\tau(m)}\right)\right]}{\rho(1_m, M_m)|S_m|(|S_m| - 1)}$$

Here, $1_m = (1, ..., 1)$ and $M_m = (M, ..., M)$.

Clearly, $ED_{X_1 \times ... \times X_m}^{max} = ED_{X_{\pi(1)} \times ... \times X_{\pi(m)}}^{max}$ for any $\pi \in S_m$, and $ED_{X_1 \times ... \times X_m}^{max} = 0$ if and only if $(X_1, ... , X_m)$ is weakly exchangeable, and the same is true for $ED_{X_1 \times ... \times X_m}^{max}$.

For the purpose of this application, we will mainly consider exchangeability of pairs of discrete random variables with values in M = {1, ..., *M*}. In this special case, since $X_1 \times X_2$ is the reflection of $X_2 \times X_1$ with respect to the line $\{(x,y) \in \mathbb{R}^2; y = x\}$, we get

$$P_{X_1 \times X_2}^{Var} = \left|Pr_{X_1 \times X_2} - Pr_{X_2 \times X_1}\right|$$

$$ED_{X_1 \times X_2}^{max} = \frac{HD_\rho(\text{supp } X_1 \times X_2, \text{supp } X_2 \times X_1)}{\rho((1,1), (M,M))}$$

$$ED_{X_1 \times X_2}^{mean} = \frac{\mathbb{E}_{X_1 \times X_2}\left[\text{dist}_\rho(X_1 \times X_2, \text{supp } X_2 \times X_1)\right]}{\rho((1,1), (M,M))}$$

**The exchangeability plot**

To illustrate the degree of weak exchangeability of a pair of discrete random variables visually we propose the exchangeability plot. The exchangeability plot for the random variables $X_1$ and $X_2$ is obtained by depicting both supp$X_1$

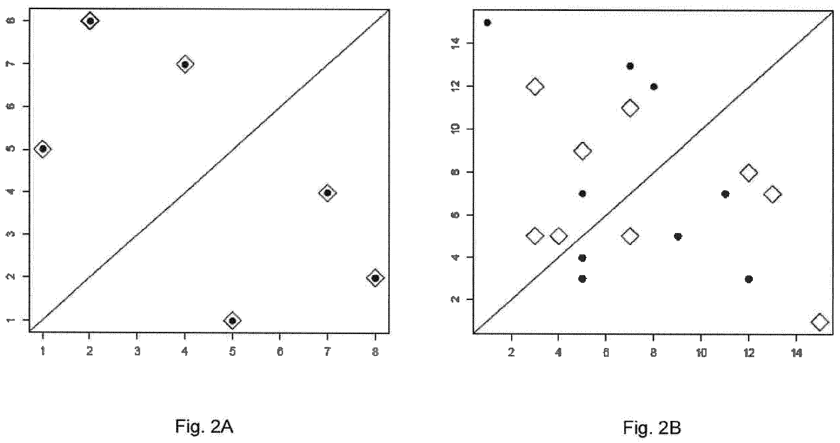

x $X_2$ and suppX$_2$ x $X_1$ in the same figure. A pair of random variables $(X_1, X_2)$ is weakly exchangeable if the two sets overlap completely. Fig. 2 shows exchangeability plots for two pairs of random variables. The pair in Fig. 2A is weakly exchangeable, while the pair in Fig. 2B is not.

**The exchangeability of genes**

In an embodiment, wherein the bioinformatics data is gene expression data we assume that we are given a universal set of M genes, denoted $\mathcal{G}$ = {$g_1$, ..., $g_M$}. Data in the form of a population (e.g. cancer patients and healthy control subjects) and a variable ranking method (e.g. a t-test contrasting the two groups in the population) is the starting point, called experiment, of the method. The sample space $\Omega$ consists of all possible rankings of the M genes, and the random variables $X_1, ... , X_M : \Omega \to$ {1,...,M} represent the ranking positions of the genes in $\mathcal{G}$. A finite set of genes $g_{i_1}, ..., g_{i_m}$ is said to be (weakly) exchangeable if the corresponding sequence of random variables $(Xi_1, ... , Xi_m)$ is (weakly) exchangeable. Intuitively, a set of genes is exchangeable if their positions in the variable ranking can be interchanged without changing the biological interpretation of the ranking.

Of course, we do not know the measures $Pr_{X_i}$ for the variables in practice, so these have to be estimated. In this description we use subsampling to generate a collection of B data sets for which we compute gene rankings. From the B gene rankings, we construct a position vector $S_i$ for each gene $g_i$ by collecting all positions of the gene in the B rankings. The elements of a position vector $S_i$ are then samples of the random variable $X_i$. Combining two position vectors $S_i$ and $S_j$ gives samples of the variables $X_i$ x $X_j$ and $X_j$ x $X_i$ which can be used to obtain estimates of $P_{X_i x X_j}^{Var}$, $ED_{X_i x X_j}^{max}$ and $ED_{X_i x X_j}^{mean}$.

We now introduce another measure of exchangeability which is especially adapted to the study of ranked gene lists. The rationale behind this measure is that we may not want two genes to obtain a small exchangeability distance if they always appear in the same order in the rankings. For example, a gene which is always ranked first is not highly exchangeable with a gene that is always ranked second, since the first gene is clearly more strongly related to the response than the second. The new measure penalizes such situations in the computation of the exchangeability distance. For a pair of random variables $(X_1, X_2)$ we define a new set-valued random variable on $\Omega$ by

$$R(X_1 \times X_2)(\omega) := \{(x,y) \in \mathbb{M} \times \mathbb{M}; \text{sign}(x - y) = (X_1(\omega) - X_2(\omega))\}.$$

*Definition 4.* The one-sided mean exchangeability distance for a pair of discrete random variables $(X_1, X_2)$ is defined by

$$oED_{X_1 \times X_2}^{mean} := \frac{\mathbb{E}_{X_1 \times X_2}\left[\text{dist}_\rho(X_1 \times X_2, \text{supp } X_2 \times X_1 \cap R(X_1 \times X_2))\right]}{\rho\big((1,2),(M-1,M)\big)}$$

if $suppX_2 x X_1 \cap R(X_1 x X_2)(\omega) \neq \emptyset$ for all $\omega \in \Omega$, and $oED_{X_1 x X_2}^{mean} = 1$ otherwise.

It is also possible to define a one-sided variant of the maximal exchangeability distance $(oED_{X_i x X_j}^{max})$ in an analogous manner. We note that due to the normalization factors introduced in the estimates of weak exchangeability, all measures introduced above attain only values in [0, 1]. This allows us to define similarity measures (exchangeability scores) for pairs of genes as follows:

$$PS_{X_i \times X_j}^{Var} = 1 - P_{X_i \times X_j}^{Var}, ES_{X_i \times X_j}^{mean} = 1 - ED_{X_i \times X_j}^{mean}, ES_{X_i \times X_j}^{max} = 1 - ED_{X_i \times X_j}^{max},$$

$$oES_{X_i \times X_j}^{mean} = 1 - oED_{X_i \times X_j}^{mean}, oES_{X_i \times X_j}^{max} = 1 - oED_{X_i \times X_j}^{max}.$$

Finally, we define normalized values of the exchangeability scores by comparing them to the corresponding values for pairs of random variables with some pre-specified distribution representing a null hypothesis of no association. In this paper, the main focus is on weak exchangeability of pairs of discrete random variables, in which case it is natural to compare to pairs of random variables $(Y_1, Y_2)$ uniformly distributed on a set $S \subseteq \mathbb{M} \times \mathbb{M}$ with cardinality equal to that of $\text{supp}X_1 x X_2$. We show only the normalization for $oES_{X_i x X_j}^{mean}$, the other scores can be normalized analogously.

*Definition 5.* The normalized one-sided mean exchangeability score for a pair of discrete random variables $(X_1, X_2)$ is defined by

$$noES_{X_1 \times X_2}^{mean} = \left( \frac{oES_{X_1 \times X_2}^{mean} - oES_{Y_1 \times Y_2}^{mean}}{1 - oES_{Y_1 \times Y_2}^{mean}} \right)_+$$

where $Y_1 x Y_2$ is a uniformly distributed random variable on a set $S \subseteq \mathbb{M} \times \mathbb{M}$ where $|S| = |\text{supp}X_1 x X_2|$, and $(a)_+ = \max(a,0)$.

We note that the measures of exchangeability depend on the number of genes in the ranking (M). For two genes having the exchangeability plot shown in Fig. 2A we get $noES_{X_1 x X_2}^{mean} = 1.0$ irrespective of the number of genes since in this case, the distance between any value of $X_1 x X_2$ and $\text{supp}X_2 x X_1$ is zero. For the exchangeability plot in Fig. 2B we obtain $noES_{X_1 x X_2}^{mean} = 0.17$ if M=15 and $noES_{X_1 x X_2}^{mean} = 0.99$ if M=1,000.

## General idea

In this section, we present a general framework for list representation and comparison, with a focus on an embodiment of the present invention applicable to analysis of biologic or bioinformatics data, and in particular analysis of gene expression data.

However, as appreciated by a person skilled in the art, the bioinformatics data may be any kind of data representing a plurality i of bioinformatics data samples of each of a plurality of measurement variables g. Preferably, the bioinformatics data is bioinformatics expression data, such as DNA microarray data, RNA-seq data or real-time PCR data; microRNA data, such as RNA microarray data, RNA-seq data or real-time PCR data; DNA methylation data, such as DNA methylation microarray data; or protein expression data, such as protein microarray data, antibody array data, 2-D gel data or LC-MS data. As is appreciate by a person skilled in the art, the context of the data is what makes the data carry biological information, hence bioinformatics data. This is the subject of the ranking method, which ultimately enables indication of a biological state.

The lists are represented as vectors in $\mathbb{R}^M$, where the entry in position i gives the contribution of gene $g_i$. The vector representation allows us to compare both ranked and unranked gene lists within the same framework, using one of the many similarity or dissimilarity measures available to compare vectors in $\mathbb{R}^M$. This is an advantage compared to existing methods for list comparison, which are specifically designed to compare certain types of lists. Our framework also provides a way to determine which genes are most important for explaining the similarity between two lists. Assume for example that some measure based on the scalar product in $\mathbb{R}^M$ is used to measure the similarity between two vectors x and y. Then the value of $x_i y_i$ is a measure of the influence of the *i*'th variable on the similarity between the two lists. Finally, ordering the genes by their weights in the vector provides a new ranking of the genes.

In an embodiment, as above, we have a universal set of M genes, $G = \{g_1, ..., g_M\}$, where the genes are indexed in a fixed (but otherwise arbitrary) fashion. The universal set can be e.g. all genes on a microarray chip. An ordered (or unordered) gene list is then an ordered (or unordered) subset of the universal set.

Let $\ell \subseteq \mathcal{G}$ denote a list. If $\ell$ is ordered and if gene $g_i$ is contained in $\ell$, we denote its position by $\pi_\ell(i)$. If $\ell$, we define $\pi_\ell(i) = 0$. For an unordered list, we let $\pi_\ell(i) := \chi_\ell(g_i)$, where $\chi_\ell$ is the characteristic function of the set $\ell$. Given a list $\ell$ we define a corresponding list matrix $G_\ell$ as the product of three basic *MxM* matrices;

$$G_\ell := A_\ell V_\ell W_\ell$$

The three basic matrices are designed to represent different characteristics of $\ell$. We call $A_\ell$ the position matrix, $V_\ell$ the exchangeability matrix and $W_\ell$ the global weight matrix. From the list matrix we form a list vector $l_\ell = ((l_\ell)_1, \ldots ,$ $(l_\ell)_M)$, by letting $(l_\ell)_i := h((G_\ell)_i)$ where $(G_\ell)_i$ is the $i$:th column of $G_\ell$ and $h: \mathbb{R}^M \to \mathbb{R}$ is a summarization function, e.g. a norm. The list vector will be used as the vector representation of the list. Once all lists of interest are represented by vectors in $\mathbb{R}^M$, we can define the similarity between them e.g. as the cosine of the angle between the corresponding list vectors, i.e.

$$s(\ell_1, \ell_2) = \frac{l_{\ell_1} \cdot l_{\ell_2}}{\|l_{\ell_1}\| \|l_{\ell_2}\|}$$

where $\bullet$ denotes the inner product in $\mathbb{R}^M$, and we can obtain a dissimilarity coefficient as

$$d(\ell_1, \ell_2) = 1 - s(\ell_1, \ell_2)$$

Choosing $A_\ell$, $V_\ell$, $W_\ell$, $h$ and the (dis)similarity coefficient on $\mathbb{R}^M$ suitably, most methods currently available for list comparison fit into this general framework.

**The position matrix**

The position matrix $A_\ell$ is defined as a diagonal matrix that contains information about the type of list (ordered or unordered) and the positions of the genes within the list. We define the diagonal elements (the position values) by $(A_\ell)_{ii} = u(r_i)$ where $r_i$ is the ranking statistic of gene $g_i$, and $u : \to \to$ is a monotone function.

For all genes not in the list, we define $(A_\ell)_{ii} = 0$. In this paper we mainly use rank-based functions, i.e. letting $(A_\ell)_{ii} = \tilde{u}(\pi_\ell(i))$ where $\tilde{u}: \mathbb{N} \to \mathbb{R}$ is a decreasing function of $\pi_\ell(i)$ if $\pi_\ell(i) > 0$, and $\tilde{u}(0) = 0$.

This means that the diagonal elements corresponding to genes in the top of the list $\ell$ are high, while the genes further down in the list obtain lower values. Furthermore, all genes not in the list have position value zero. We note that in some cases, other choices of position values may be better suited for unordered lists, where it may be desirable to give the genes different weights, e.g. depending on some external criterion, even though there is no specified ordering.

**The exchangeability matrix**

The exchangeability matrix $V_\ell$ carries information about the exchangeability between the genes in $\mathcal{G}$ in the specific experiment giving the list $\ell$. In an embodiment, we define the entry $(V_\ell)_{ij}$ to be the estimated normalized one-sided mean exchangeability score of $g_i$ and $g_j$ (i.e. $\widehat{noES^{mean}_{X_i \times X_j}}$), so the diagonal elements are always 1. If $V_\ell$ is diagonal, i.e. $V_\ell = I_M$, then the only non-zero elements in the list vector are those corresponding to genes that are actually contained in $\ell$ and consequently only the genes that are present in the list affect its vector representation. However, if $V_\ell$ is not diagonal, there is a possibility that the vector representation of the list is extended, i.e. that it contains non-zero entries for genes which are not themselves present in the list, but are exchangeable with some gene in the list. The (absolute) weight of a gene in the list can also be increased if it is highly exchangeable with a gene with a higher (absolute) position value, since the high exchangeability indicates that the genes could as well have switched positions without changing the interpretation of the list.

We note that the general framework for list representation supports any matrix of gene similarities in the place of $V_\ell$. Thus, in an embodiment, $V_\ell$ could be defined from some kind of expert biological knowledge, e.g. concerning which genes are related to the same biological function. This could be used for example when comparing lists from different experiments to each other. In another embodiment, the positive part of the correlation matrix is used in place of $V_\ell$ since a high correlation between the expression levels of two genes is often considered to indicate similar biological functions of the genes.

**The global weight matrix**

The global weight matrix $W_\ell$ is a diagonal matrix that permits weighting the influence of the genes differently, depending on some informativeness or reliability estimate. For example, we may wish to downweight the influence of a gene that has a high probability to be present in an arbitrarily chosen list, since this gene is unspecific and may not give much relevant information about the similarity between a pair of lists.

The specific embodiment following description focuses on an embodiment of the present invention applicable to analysis of biologic data, and in particular analysis of gene expression data. However, it will be appreciated that the invention is not limited to this application but may be applied to many other data including for example meteorology and astronomy.

**Specific embodiment**

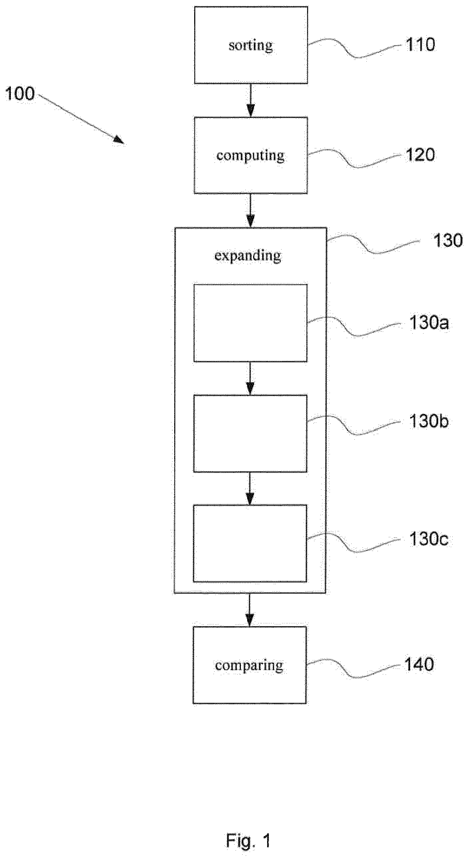

In the embodiment according to Fig. 1, bioinformatics data comprising a plurality of samples was obtained from a microarray study of lung adenocarcinoma patients, as provided in Bhattacharjee, A., Richards, W.G., Staunton, J., Li, C., Monti, S., Vasa, P., Ladd, C., Beheshti, J., Bueno, R., Gillette, M., Loda, M., Weber, G., Mark, E. J., Lander, E. S., Wong, W., Johnson, B. E., Golub, T. R., Sugarbaker, D. J., and Meyerson, M. (2001), Classification of human lung carcinomas by mRNA expression profiling reveals distinct adenocarcinoma subclasses. A ranking method, signal-to-noise ratio, was also provided.

The abovementioned bioinformatics data set consists of gene expression profiles from 31 patients with "good outcome" and 31 patients with "poor outcome". The data set was downloaded from http://www.broadinstitute.org/gsea/datasets.jsp (Lung_Boston_collapsed.gct), on January 11, 2011. In the downloaded file, original Affymetrix probe set IDs have been replaced with gene symbols and all probe sets matching to the same gene have been collapsed into one variable.

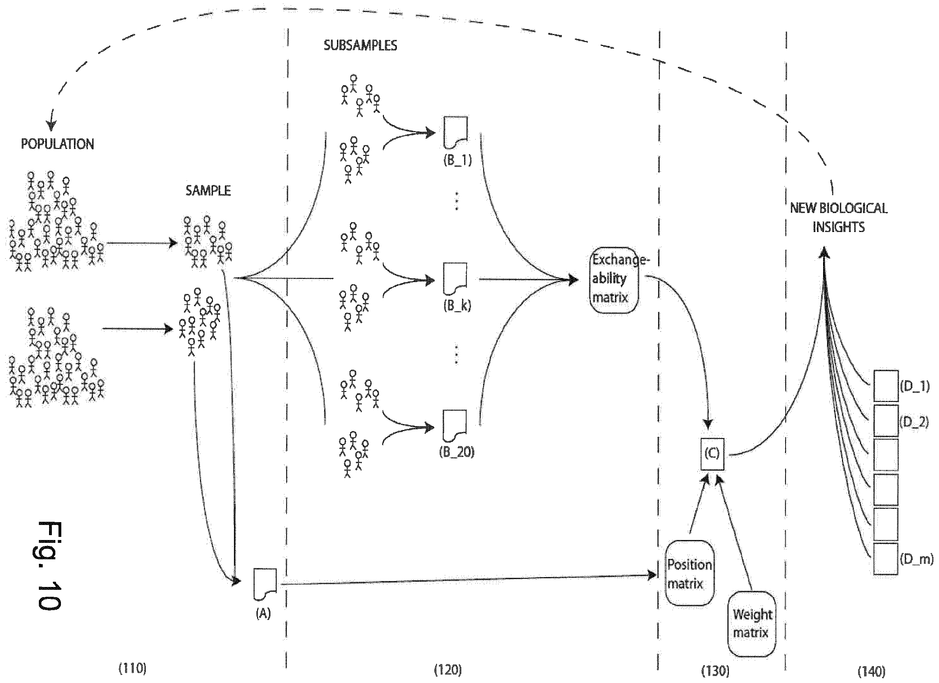

The embodiment according to Fig. 1 is further visualized in Fig. 10. Here, the large group of persons on the left side indicates the population that we are interested in studying. For example, it can be patients with breast cancer and healthy controls, or lung cancer patients with good or poor prognosis, respectively. It can also be for example rats treated with different drugs, or a homogeneous collection of plants that we want to characterize. From the population, we draw a sample of patients, rats, etc. to include in the experimental study. Reference (A) represents the ranked list of variables obtained in step 110.

In Fig. 10, step 120 corresponds to the exchangeability computation step. The subsamples are subcollections of the whole sample. As will be appreciated by a person skilled in the art, the right way to construct them depends on the application, specifically it depends on what we want to stabilize the ranked list with respect to. The subsamples are used to create ranked variable lists (B_1 to B_20). From these ranked variable lists we compute the exchangeabilities among the variables. These are collected into the exchangeability matrix.

Step 130 represents the expansion step. From the original ranked list (A) we compute a position matrix for the variables. We are also given a weight matrix (possibly the identity matrix) that contains prior knowledge about the reliability of the variables. The position matrix, the exchangeability matrix, and the weight matrix are multiplied and the values in the resulting matrix are summarized into an expanded list vector (C).

In step 140 the expanded list vector (C) is compared to other list vectors (D_1 to D_m). These list vectors can be obtained in the same way as (C) or in any other way. An important special case is when the list vectors are used to represent gene sets. In this case, the list vectors (D_1) to (D m) are binary vectors. By comparing the expanded list vector (C) to the list vectors (D_1) to (D m) new biological insights concerning the population can be generated. The goal of the process 110-140 is to obtain a stabilization effect so that these biological insights are not sensitive to the specific choice of the sample.

In the data set, 62 patient observations (31 with "good" outcome and 31 with "poor" outcome) thus gave 62 samples in form of observation vectors, comprising 5,217 measurement variables g, i.e. studied genes.

Signal-to-noise ratio (SNR) for each variable in the data was computed as

$$SNR = \frac{m_{poor} - m_{good}}{\sigma_{poor} + \sigma_{good}}$$

where $m_{poor}$ and $m_{good}$ is the mean value of the expression values in the "poor outcome" and "good outcome" group, respectively and $\sigma_{poor}$ and $\sigma_{good}$ is the standard deviation of the expression values in the "poor outcome" and "good outcome" group, respectively. The signal-to-noise ratios provide information about the biological importance of the variables with respect to the current task.

The variables were then sorted 110 by decreasing value of the computed statistic. In this way, each variable i obtains a position in the ranking. The variable with the highest (positive) SNR value obtained position 1, and the variable with the lowest (most negative) SNR value obtained position 5217. The experimental list is now the set of variables sorted in the indicated order, i.e. an ordered list $\ell$. The goal is to analyze the obtained list to enable robust biological conclusions. In Fig. 10, the experimental ranked list is indicated by (A). The top-ranked genes in this ordered list are DBP, ALDH2, PBP, CYP4B1, CAT, EEF1A1, KIAA0256, IL12RB1, ATP5A1 and RPLP1. Table 1 shows the top-ranked genes in this ordered list. The ranking of the variables by their SNRs is unstable and sensitive to the initial choice of patients to include in the study. Thus, in order to provide biological insights of a general character, it needs to be stabilized.

*Table 1.* The top-ranked genes in the ordered list according to an embodiment.

| No. | Gene | No. | Gene | No. | Gene |
|---|---|---|---|---|---|
| 1 | DBP | 18 | CCR7 | 35 | HABP2 |
| 2 | ALDH2 | 19 | SDHB | 36 | TIMM44 |
| 3 | PBP | 20 | STAT3 | 37 | LTB4R |
| 4 | CYP4B1 | 21 | PLA2G2A | 38 | GPC3 |
| 5 | CAT | 22 | SCNN1B | 39 | EFNA1 |
| 6 | EEF1A1 | 23 | SLC14A2 | 40 | SGK |
| 7 | KIAA0256 | 24 | PBXIP1 | 41 | MUF1 |
| 8 | IL12RB1 | 25 | MVP | 42 | PKLR |
| 9 | ATP5A1 | 26 | GPD1L | 43 | DDOST |
| 10 | RPLP1 | 27 | SNX1 | 44 | IL11RA |
| 11 | CYFIP2 | 28 | CLU | 45 | EMD |
| 12 | ALDH9A1 | 29 | RPS28 | 46 | EBAF |
| 13 | CR2 | 30 | PLD3 | 47 | CD37 |
| 14 | AZGP1 | 31 | CYP27A1 | 48 | CEBPA |
| 15 | GSN | 32 | ESD | 49 | RPL11 |
| 16 | COL9A2 | 33 | TIAM1 | 50 | LMO3 |
| 17 | ARHGDIG | 34 | FHIT | | |

Next, exchangeabilities between each pair of variables were computed 120 and collected in a 5217 x 5217 matrix. The exchangeabilities were computed in two steps.

Firstly, position vectors were computed by generating 20 slightly modified data sets by sub sampling the original data set, each time keeping 2/3 of the patients (randomly chosen) from each outcome group. For each modified data set, a signal-to-noise ratio (SNR) is computed for each variable as shown above, and used to rank the variables. In Fig. 10, the ranked lists are indicated by B_1,...,B_20. Table 2 shows the top-ranked genes in three of the ranked lists obtained in this way. Note that there are considerable discrepancies between the top-ranked genes in these lists, further indicating that the variable ranking is highly dependent on the choice of included samples and needs stabilization.

*Table 2.* The top-ranked genes in three of the ranked lists obtained in an embodiment.

| No. | Gene ranking 1 | Gene ranking 2 | Gene ranking 3 |
|---|---|---|---|
| 1 | CAT | CLU | IL12RB1 |
| 2 | EEF1A1 | PBP | CCL25 |
| 3 | PBP | ALDH2 | DBP |
| 4 | CDW52 | FOLR1 | PBP |
| 5 | SLC14A2 | KIF1A | COL18A1 |
| 6 | GPC3 | CAT | C21orf33 |
| 7 | DBP | DCL-1 | CAT |
| 8 | TRB@ | SLC14A2 | FAAH |
| 9 | IER2 | CR2 | SLC6A11 |
| 10 | GPT | IL12RB1 | AGER |
| 11 | CR2 | RPLP1 | ITGA2B |
| 12 | LMO3 | CEBPA | POMZP3 |
| 13 | COL9A2 | EEF1A1 | BMP2 |
| 14 | CCR7 | RPS28 | ARHGDIG |

(continued)

| No. | Gene ranking 1 | Gene ranking 2 | Gene ranking 3 |
|---|---|---|---|
| 15 | ALDH9A1 | TAF12 | POLR3D |
| 16 | SGK | CTSE | EBAF |
| 17 | PLA2G2A | RNASE1 | CEBPA |
| 18 | UBE2D2 | TCL1A | GPD1L |
| 19 | PFAAP5 | PGC | C9orf61 |
| 20 | FOSB | FAAH | GPC3 |
| 21 | SYBL1 | PLA2G2A | PLA2G2A |
| 22 | OSIL | PLD3 | PRKR |
| 23 | TIAM1 | DBP | PPY |
| 24 | RPL10A | STAT3 | PROS1 |
| 25 | ESD | GPD1Ö | RARA |
| 26 | SDHB | ALDH9A1 | TRPC2 |
| 27 | HBB | IGFBP6 | SAH |
| 28 | IL12RB1 | SCNN1B | MAP3K11 |
| 29 | RPL11 | HP | LTB4R |
| 30 | CYP27A1 | CLCN1 | DJ462023.2 |
| 31 | AZGP1 | NINJ1 | SLC25A4 |
| 32 | RPLP1 | CD37 | OPRK1 |
| 33 | CRYAB | LMO3 | PNPLA4 |
| 34 | COG2 | CYFIP2 | OSIL |
| 35 | ADK | NXF1 | ALDH2 |
| 36 | ATF3 | ACADSB | LRRC6 |
| 37 | EMD | NME3 | GPT |
| 38 | RPS28 | CYP2C19 | EEF1A1 |
| 39 | EBI2 | EPHX1 | CHP |
| 40 | ATP5A1 | ORM1 | PCCA |
| 41 | EMP1 | CCR7 | SDHB |
| 42 | FUCA1 | PHOX2B | CYP4B1 |
| 43 | PEPD | CYP4B1 | SCGB1A1 |
| 44 | SCNN1B | GSN | NR3C2 |
| 45 | ALDH2 | AZGP1 | COL9A2 |
| 46 | GUK1 | DSCR1L1 | NFATC1 |
| 47 | TNNC1 | MADH7 | TAF12 |
| 48 | PROL3 | ATP5A1 | MVP |
| 49 | GPD1L | SSTR3 | GPA33 |
| 50 | CCL25 | POLR3D | PFKL |

The positions that a variable obtains in the 20 rankings are collected in a position vector for the variable. The position vector for variable $i$ is denoted by $S_i$ ($1 \le i \le 5217$).

Secondly, for each pair of variables, the exchangeability score was estimated according to the following. For each pair of variables the joint position vectors were formed as:

$$S_{ixj} = \left\{ \left( (S_i)_k, (S_j)_k \right) \right\}_{k=1}^{20}$$

and

$$S_{jxi} = \left\{ \left( (S_j)_k, (S_i)_k \right) \right\}_{k=1}^{20}$$

These vectors describe the observed positions for the ordered pairs of variables. Then, the one-sided mean exchangeability distance between the two variables was estimated by

$$\dot{o}ED_{X_i x X_j}^{mean} = \frac{\sum_{k=1}^{20} \frac{1}{20} dist_\rho \left( \left\{ \left( (S_i)_k, (S_j)_k \right) \right\}, \left\{ \left( (S_j)_v, (S_i)_v \right) \right\}_{v=1}^{20} \cap R_{ij}(k) \right)}{\rho((1,2),(5216,5217))}$$

In this equation, $\rho$ is the standard Euclidean metric in $\mathbb{R}^2$ and, for two sets A and $B$ in $\mathbb{R}^2$, $dist_\rho(A,B) = \min_{a \in A, b \in B} \rho(a,b)$ were defined.

Finally,

$R_{ij}(k) = \{(x,y) \in \mathbb{M} \times \mathbb{M}; sign(x - y) = sign ((S_i)_k - (S_j)_k)\}$ denotes the points in $\{1,...5217\} \times \{1,...5217\}$ that are on the same side of the diagonal as the $k$'th point. In case $\left\{ \left( (S_j)_v, (S_i)_v \right) \right\}_{v=1}^{B} \cap R_{ij}(k) = 0$ for some $k$, the one-sided mean exchangeability distance between the two variables was defined to be 1.

From the one-sided mean exchangeability distance a one-sided exchangeability score was formed by

$$\dot{o}ES_{X_i x X_j}^{mean} = 1 - oED_{X_i x X_j}^{mean}.$$

The one-sided mean exchangeability score is normalized to give a normalized one-sided mean exchangeability score, by

$$\dot{n}oES_{X_i x X_j}^{mean} = \max \left( 0, \frac{\dot{o}ES_{X_i x X_j}^{mean} - \dot{o}ES_{Y_i x Y_j}^{mean}}{1 - \dot{o}ES_{Y_i x Y_j}^{mean}} \right).$$

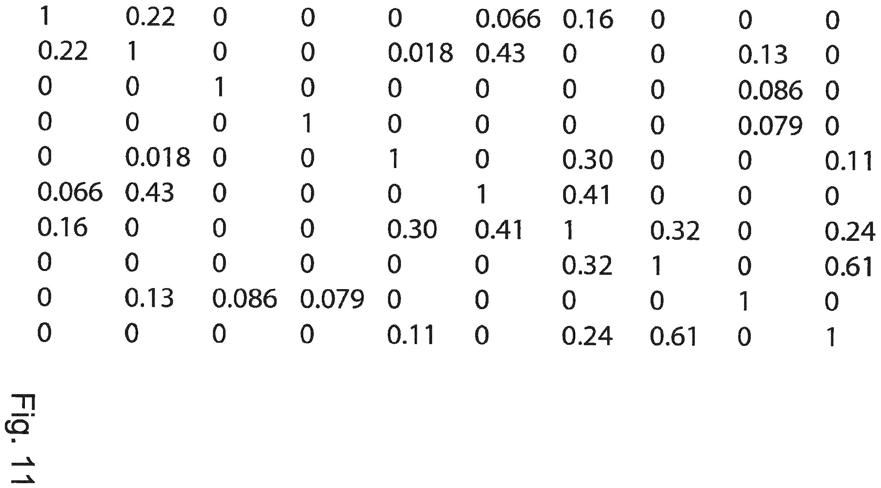

The normalization constant $\dot{o}ES_{Y_i x Y_j}^{mean}$ is the estimated value of the one-sided mean exchangeability score for variable pairs uniformly distributed on a set $S \subset \{1,...5217\} \times \{1.... 5217\}$ with 20 elements. The estimated value $\dot{n}oES_{X_i x X_j}^{mean}$ is then put in position $(i,j)$ in the exchangeability matrix $V_\ell$. A part of the exchangeability matrix according to an embodiment, containing the exchangeabilities among the first 10 variables is shown in Fig. 11. Note that the exchangeability between a variable and itself is always 1.

Next, the ordered list $\ell$ of the data is expanded 130, thus combining the computed exchangeabilities with the information about the position of the variables in the experimental list to obtain a representation of the experimental list as a vector in $\mathbb{R}^{5217}$. In an embodiment, this is done in several steps.

First, a diagonal position matrix $A_\ell$ is computed 130a, where the entry in the $i$'th row and column is derived from the position of the $i$'th variable in the list of experimental data. For all variables with a positive SNR as computed above (i.e. the variables in the top of the list),

$$A_{ii} = \frac{350}{(\pi(i)-1)^2+350}$$

is defined where $\pi(i)$ is the position of variable $i$ in the list. For variables with a negative SNR,

$$A_{ii} = -\frac{350}{(5217 - \pi(i))^2 + 350}$$

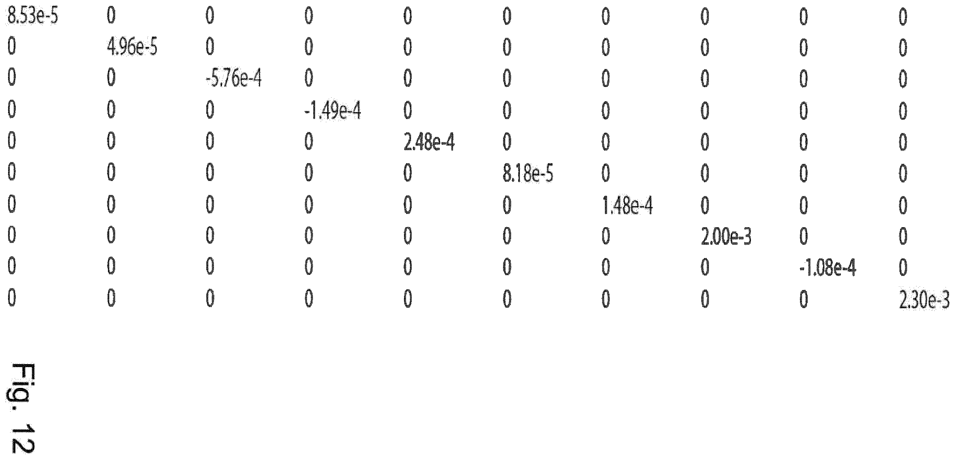

is defined. Fig. 12 shows the part of the position matrix corresponding to the first 10 variables according to an embodiment. Note that some variables have positive position values, indicating that they have positive SNRs, and others have negative position values.

Second, a global weight matrix $W_\ell$ is computed 130b, which permits inclusion of a weight for each variable, which can be used to put more emphasis on e.g. genes that are known to be reliably measured. Since there is no such information in this example, the global weight matrix is taken to be the identity matrix.

Third, an expanded experimental list vector $l_\ell$ is computed 130c. The expanded list vector $l_\ell$ is taken as the vector representation of the experimental list in $\mathbb{R}^{5217}$, according to the formula:

$$l_\ell = ((l_\ell)_1, \ldots, (l_\ell)_M)$$

by letting

$$(l_\ell)_i = h((G_\ell)_i)$$

where $G_\ell = A_\ell V_\ell W_\ell$, and where $(G_\ell)_i$ is the $i$'th column of $G_\ell$ and h: $\mathbb{R}^M \to \mathbb{R}$ is defined by letting the $i$'th element of the list vector be the entry with the largest magnitude from column i in the list matrix. The list vector is called expanded since the value in each position is constructed using information from all the variables in the data set. Fig. 12 shows the first 10 elements in the expanded list vector according to an embodiment. These elements indicate the stabilized importance of the first 10 genes in the considered application. Table 3 shows the 50 genes corresponding to the highest values in the expanded list matrix. This ranking can be interpreted as a stabilized variant of the original gene ranking, cf. (A) in Fig. 10. Compared to Table 1, some genes (e.g. IL12RB1, RPLP1 and PPP3CB) are ranked higher in the stabilized ranking, indicating that in this more general ranking, these genes obtain increased importance for the discrimination between good and poor outcome lung cancer patients. Conversely, some genes (e.g. CAT, EEF1A1 and ATP5A1) are ranked higher in the original ranking than in the stabilized ranking. This suggests that their top-ranking positions in the original ranking could be an effect of the specific sample and not as suitable to generalize to the population.

*Table 3*. The 50 genes corresponding to the highest values in the expanded list matrix according to an embodiment.

| No. | Gene | No. | Gene | No. | Gene |
|-----|------|-----|------|-----|------|
| 1 | DBP | 18 | LAF4 | 35 | SDHB |
| 2 | ALDH2 | 19 | BIRC5 | 36 | SHMT1 |
| 3 | PBP | 20 | ESD | 37 | MGC17330 |
| 4 | CYP4B1 | 21 | MIPEP | 38 | MSTP9 |
| 5 | IL12RB1 | 22 | MVP | 39 | DSCR1 |
| 6 | CAT | 23 | FGFR2 | 40 | AZGP1 |
| 7 | KIAA0256 | 24 | PROL3 | 41 | CCR7 |
| 8 | EEF1A1 | 25 | COG2 | 42 | ATP5A1 |
| 9 | RPLP1 | 26 | APOA4 | 43 | ABAT |
| 10 | PPP3CB | 27 | CYFIP2 | 44 | OSIL |
| 11 | PLA2G2A | 28 | SCNN1B | 45 | UCN |
| 12 | PCSK2 | 29 | CR2 | 46 | RPS28 |
| 13 | IL11RA | 30 | PKLR | 47 | CLCN4 |
| 14 | MGP | 31 | FHIT | 48 | PHOX2B |
| 15 | PEPD | 32 | CRYAB | 49 | MAPK11 |
| 16 | LTB4R | 33 | ALDH9A1 | 50 | HABP2 |
| 17 | TIAM1 | 34 | PBXIP1 | | |

Finally, the expanded experimental list vector $l_\ell$ is compared 140 to another list vector comprising a plurality i of measurement variables g, i.e. the gene sets.

To compare the experimental list vector $l_\ell$ to the gene sets from MSigDB, vector representations of the gene sets are constructed as well. Since the gene sets are not generated directly from an experiment as the experimental list, there is not enough information to construct the exchangeability matrix as for the experimental list. Moreover, the gene sets are not ranked, i.e. the elements in a gene set do not have a specific order. Each gene set is therefore represented by a vector with 5217 elements, where the elements corresponding to the genes in the gene set are set to 1 and the others to 0. Thus, the list vector for a gene set is similar to the list vector in Fig. 12, but the elements are either 0 or 1, indicating inclusion or exclusion of the corresponding gene in the gene set.

Now, both the experimental list and the gene sets are represented by vectors of length 5217. A dissimilarity score is computed between two vectors by taking 1 minus the cosine of the angle between them, which is easily computed directly from the vectors. The dissimilarity score lies in the interval [0,2], where 0 is obtained only for identical list vectors. The output from this step is a list of dissimilarity scores (one for each gene set). The gene sets with the smallest dissimilarity score are most closely related to the genes in the top of the list (i.e. to the genes over expressed in the group with poor outcome relative to the group with good outcome), while the gene sets with the largest dissimilarity score are most closely related to the genes in the bottom of the list (i.e. to the genes over expressed in the group with good outcome relative to the group with poor outcome). In this way, conclusions can be drawn concerning which gene sets (and thereby which cellular functions) are most closely related to the two groups of observations.

These conclusions may be important for different therapeutical and/or diagnostical reasons, or for research purposes. The gene sets that are closest to the experimental list indicate e.g. biological pathways that are associated with the outcome of the patients. Thus, the results can give rise to new biological insights concerning the population, since a determination of a specific relationship between samples is enabled, which relationship is indicative of a biological state, and which enables improved analysis of the biological state.

**Reference Example 1**

We will now estimate the robustness of the biological conclusions drawn in the abovementioned embodiment as described above. The effect of the stabilization will be estimated by repeating the same analysis without introducing the exchangeabilities, and both methods will be contrasted with what is obtained from Gene Set Enrichment Analysis (GSEA) (Subramanian et al., 2005). The robustness is estimated in the following way:

First, we generate ten slightly modified data sets from the original data through bootstrapping, i.e. sampling 31 observations from each outcome group ("good" and "poor") with replacement. Hence, some patient may be included more than once in the same modified data set.

Second, we compute two list vectors for each bootstrapped data set. The first list vector, the expanded list vector, is computed by sorting 110, computing 120 an exchangeability score and expanding 130, as described above. The second list vector, the non-expanded list vector, is obtained in the same way but using the identity matrix instead of the exchangeability matrix $V_\ell$. In this way, each entry in the list vector is affected only by the position of the corresponding variable.

Third, we compute the distances between the two list vectors and each of the gene sets by comparing 140 them for each of the ten bootstrapped data sets. The ten gene sets that have the shortest distance to the respective list vector and the ten gene sets that have the longest distance to the respective list vector are stored. For each pair of bootstrapped data sets, we compute the overlap between the collections of gene sets with shortest distances as well as the overlap between the collections of gene sets with longest distances to each of the two types of experimental list vectors (expanded and non-expanded).

For each of the ten bootstrapped data sets, we also apply GSEA using the R package provided by the authors at http://www.broadinstitute.org/gsea/downloads.jsp. For each data set we find the gene sets most related to each of the outcome groups and then we compute the overlap of these collections of gene sets for each pair of bootstrapped data sets. The GSEA algorithm uses a weighted Kolmogorov-Smirnov statistic to define an enrichment score (ES) for each gene set. These are normalized to eliminate the possible effect of the size of the gene sets, yielding normalized enrichment scores (NES) for the gene sets (denoted GSEANES).

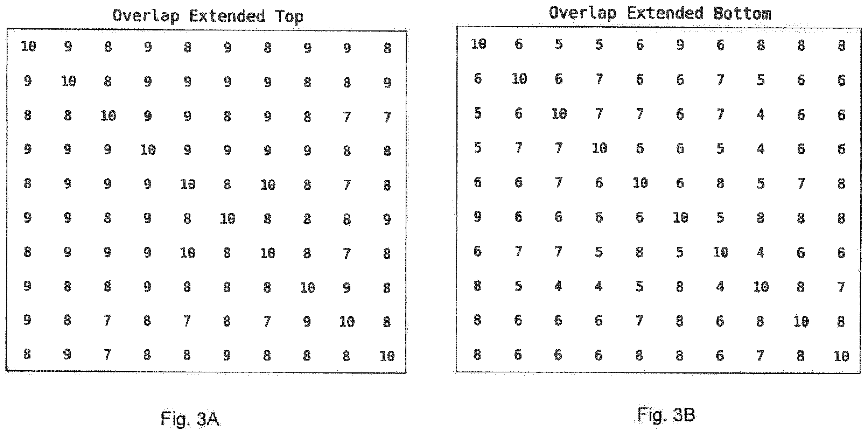

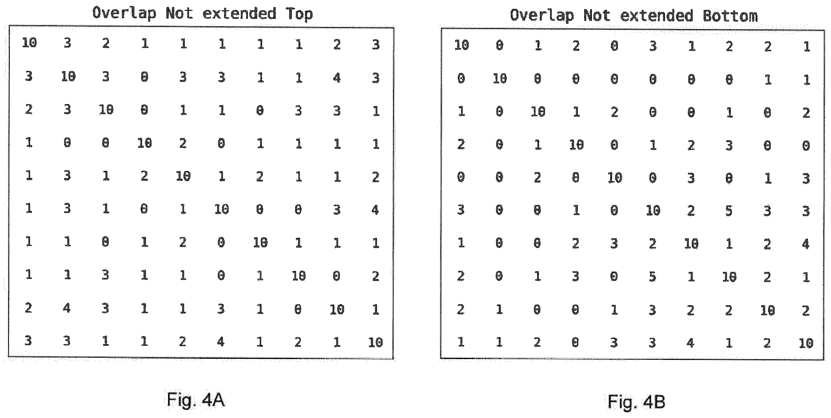

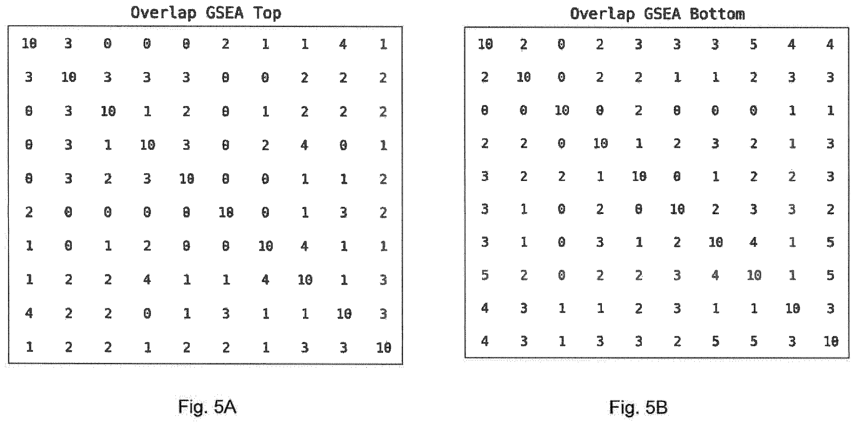

The result is seen in Figs 3-5. For each gene set collection (related to top (A), i.e. poor outcome, and bottom (B), i.e. good outcome, of the experimental list, respectively), the figures show the size of the overlap between the extracted gene sets for any two of the bootstrapped data sets. Fig. 3 is the result according to an embodiment of the invention, Fig. 4 is the result without expanding 130 the list and Fig. 5 is the result obtained using GSEA. The numbers represent degree of overlap (1-10, where 10 is maximum overlap). As can be seen, the robustness of the gene set rankings obtaining by comparing the expanded list vectors in this respect is remarkably high compared to that of the other two methods meaning that sampling variations have a much smaller impact on the resulting biological conclusions.

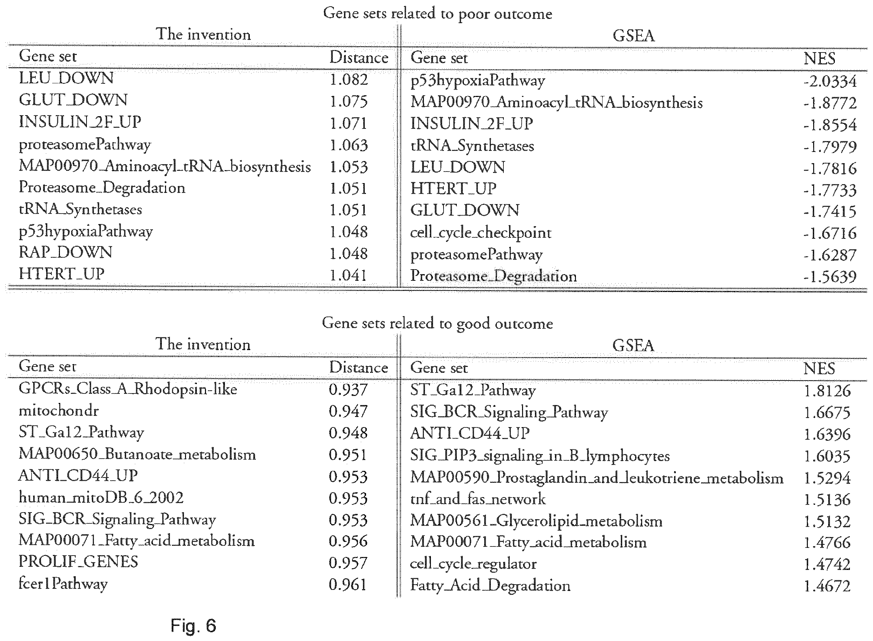

The gene sets are ranked by the distance to the extended list vector. By studying the gene sets with the shortest (or longest, respectively) distance to the experimental list we find the biological processes most related to the experimental contrast. Fig. 6 gives the closest and farthest gene sets with the method according to an embodiment of the invention "The invention" as well as the genesets with the largest positive and negative normalized enrichment score from GSEA, "GSEA". These are computed using the R implementation of the GSEA algorithm.

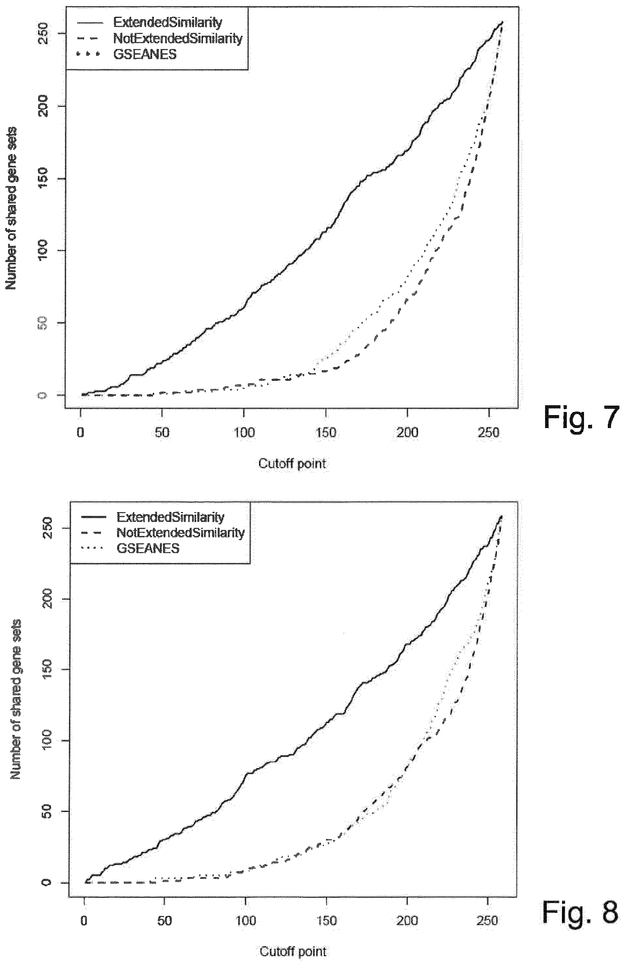

For all ranking metrics we also generate concordance plots for the gene set rankings obtained by ordering the gene sets by decreasing or increasing values of the respective ranking statistic, i.e. taking the direction of the association with the response into account. For each k the concordance plot shows the number of gene sets which arc among the top-k ranked gene sets in all boot strapped data sets. The concordance plot where top gene sets are those positively associated with poor outcome is shown in Fig. 7, and the concordance plots for the rankings where the gene sets positively associated to good outcome are ranked in the top is shown in Fig. 8. In both plots, the number of shared gene sets is on the y axis and cutoff point is on the x axis.

In both Fig. 7 and 8, it is seen that the gene set rankings obtaining by comparing the expanded list vectors, represented by a line (ExtendedSimilarity) are more stable than the other two methods, GSEA represented by dotted line (GSEANES) and unexpanded list represented by a dashed line (NotExtendedSimilarity).

Thus, the ranking metrics based on the similarity to the extended list vector consistently provide reproducible rankings.

**Reference Example 2**

Even if stability is a tractable property of a gene list from an experiment, it is not the only one. We can obtain a perfectly robust gene list by always assigning each gene the same position, but the resulting list is useless from the point of view of biological interpretation. In this part we extract gene lists in three different ways and compare the biological information contained in the top- and bottom-ranked genes in each list.

We compute three gene lists as follows. First, the non-expanded gene list which is the original list obtained by sorting the genes according to the SNR values. Then, the expanded gene list which is computed from the expanded list vector (obtained as described above) by sorting the genes according to their value in the expanded list vector. Finally, we compute an aggregated gene list by computing the median position in the position vector across 100 rankings from subsampled data sets for each gene and sorting the genes according to this, with the gene with lowest mean position in the top.

For each of the three gene lists, we extract a subset of the original data set consisting only of the top-k and bottom-k genes (for different choices of k). This small data set is fed into a centroid classifier (Schölkopf and Smola (2002): Learning with kernels, p. 4-5) and we estimate the classification ability of the subset by means of tenfold cross-validation. Table 1 shows the estimated classification accuracy for top and bottom genes from the expanded gene list, the non-expanded gene list and the aggregated gene list, as well as the mean classification accuracy for 20 random gene lists. The best performing subset for each k is highlighted in bold.

Apparently, the increased stability does not come at the price of reduced biological meaning since the top and bottom genes from the expanded gene list perform best in the classification task.

*Table 4*. The estimated classification accuracy for top and bottom genes of different lists.

|  | $k = 1$ | $k = 10$ | $k = 30$ | $k = 100$ |
|---|---|---|---|---|
| Extended | 0.344 | **0.774** | **0.837** | **0.832** |
| Non-extended | 0.344 | 0.583 | 0.606 | 0.566 |
| Aggregated | 0.410 | 0.565 | 0.617 | 0.566 |
| Random | **0.464** | 0.489 | 0.473 | 0.478 |

**Implementation**

In an embodiment, a computer program product comprising computer program code means for executing the method according to some embodiments, when said computer program code means are run by an electronic device having computer capabilities.

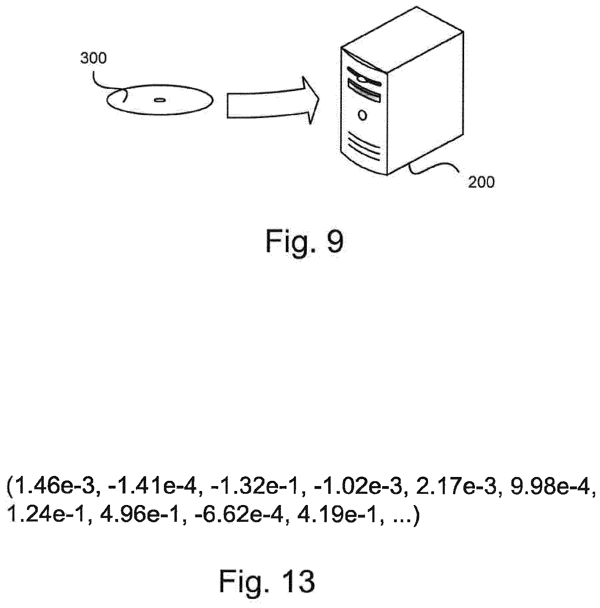

In an embodiment according to Fig. 9, a computer readable medium 300 is disclosed, having stored thereon a computer program product comprising computer program code means for executing the method according some embodiments, when said computer program code means are run by an electronic device 200 having computer capabilities.

Computer program, software program, program product, or software, in the present context mean any expression, in any programming language, code or notation, of a set of instructions intended to cause a system having computer processing capabilities to perform a particular function directly after conversion to another language, code or notation.

The electronic device 200 having computer capabilities may be any device normally used for performing the involved tasks, e.g. a hardware, such as a processor with a memory. The processor may be any of variety of processors, such as Intel or AMD processors, CPUs, microprocessors, Programmable Intelligent Computer (PIC) microcontrollers, Digital Signal Processors (DSP), etc. However, the scope of the invention is not limited to these specific processors. The memory may be any memory capable of storing information, such as Random Access Memories (RAM) such as, Double Density RAM (DDR, DDR2), Single Density RAM (SDRAM), Static RAM (SRAM), Dynamic RAM (DRAM), Video RAM (VRAM), etc. The memory may also be a FLASH memory such as a USB, Compact Flash, SmartMedia, MMC memory, MemoryStick, SD Card, MiniSD, MicroSD, xD Card, TransFlash, and MicroDrive memory etc. However, the scope of the invention is not limited to these specific memories.

The invention may be implemented in any suitable form including hardware, software, firmware or any combination of these. However, preferably, the invention is implemented as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors.

**Claims**

1. A computer-implemented method (100) for identifying a set having a relationship between a plurality $i$ of bioinformatics data samples of each of a plurality of measurement variables g, based on a ranking method for sorting said data, said relationship being indicative of a biological state, said computer-implemented method comprising

   sorting (110) the bioinformatics data into an ordered list $\ell$ according to the ranking provided by the ranking method; **characterized in that** the computer-implemented method further comprises computing (120) an exchangeability score of pairs of variables of the ordered bioinformatics data, wherein the exchangeability score is a similarity measure for the variables, and the subsets of variables are pairs of variables with an exchangeability score exceeding a predefined threshold value, resulting in an exchangeability matrix $V_\ell$ that carries information about the exchangeability between each of the plurality of measurement variables g such that the entry $(V_\ell)_{ij}$ of the exchangeability matrix $V_\ell$ is the exchangeability score of measurement variables $g_i$ and $g_j$;
   expanding (130) the ordered list $\ell$ of the data by multiplying the computed exchangeability matrix $V_\ell$ with a computed diagonal matrix $A_\ell$ comprising information of the position of the variables in the ordered list $\ell$ to obtain a representation of the ordered list as an expanded experimental list vector $l_\ell$; and
   comparing (140) the expanded experimental list vector $l_\ell$ with another list vector comprising a plurality of measurement variables g by computing a dissimilarity score between the expanded experimental list vector $l_\ell$ and the list vector comprising a plurality of measurement variables g resulting in a list of dissimilarity scores, each dissimilarity score corresponding to a specific set of measurement variables g; and
   determining the relationship between the samples by identifying the specific set of measurement variables g having the smallest dissimilarity score and/or the specific set of measurement variables g having the largest dissimilarity score, thereby reducing the plurality $i$ into an identified set of data samples being indicative of the biological state.

2. The computer-implemented method according to claim 1, further performing the comparing (140) at least twice with different list vectors comprising a plurality of measurement variables g and selecting a list vector with the lowest and/or highest dissimilarity score, thereby reducing the plurality $i$ into an identified set of data samples being highly indicative of the biological state.

3. The computer-implemented method according to claim 1 or 2, wherein the expanding (130) comprises the steps computing (130a) the diagonal position matrix $A_\ell$, by defining diagonal elements as:

$$(A_\ell)_{ii} = u(r_i)$$

   where $r_i$ is the ranking statistic of variable i used in sorting (110) the data into an ordered list $\ell$, and $u : \to \to$ is a monotone function;

   computing (130b) a diagonal global weight matrix $W_\ell$ by weighting the data;
   computing (130c) the expanded experimental list vector $l_\ell$ according to a function:

$$f(A_\ell, V_\ell, W_\ell) = l_\ell.$$

4. The computer-implemented method according to claim 3, wherein computing (130c) an expanded experimental list vector $l_\ell$ is done according to the formula:

$$l_\ell = ((l_\ell)_1, \dots, (l_\ell)_M)$$

by letting

$$(l_\ell)_i = h((G_\ell)_i)$$

where $G_\ell = A_\ell V_\ell W_\ell$; and $(G_\ell)_i$ is the $i$:th column of $G_\ell$ and $h: \mathbb{R}^M \to \mathbb{R}$.

5. The computer-implemented method according any of the preceding claims, wherein the ranking method is signal-to-noise ratio (SNR), fold change of average expression value, t-test, ANOVA, and non-parametric tests.

6. The computer-implemented method according to any of the preceding claims, wherein the computing (120) an exchangeability score of each pair of variables is total exchangeability variation score, PS, mean exchangeability score, ES(mean), maximal exchangeability score, ES(max), one-sided mean exchangeability score, oES(mean), one-sided maximal exchangeability score, oES(max), or normalized variants thereof.

7. The method according to any of the preceding claims, wherein the bioinformatics data is gene expression data, microRNA data, DNA methylation data, or protein expression data.

8. A computer program product comprising computer program code means for executing the method according to any of the claims 1 to 7 when said computer program code means are run by an electronic device (200) having computer capabilities.

9. A computer readable medium (300) having stored thereon a computer program product comprising computer program code that causes an electronic device (200) having computer abilities to execute the method according to any of the claims 1 to 7 when said computer program code is loaded into and executed by a controller of the electronic device (200).

10. Apparatus for identifying a set having a relationship between a plurality $i$ of bioinformatics data samples of each of a plurality of measurement variables g, based on a ranking method for sorting said data, said relationship being indicative of a biological state, said apparatus comprising a memory for storing data and instructions and a controller, wherein said controller is configured to

sort (110) the bioinformatics data into an ordered list $\ell$ according to the ranking provided by the ranking method; **characterized in that** the controller is further configured to
compute (120) an exchangeability score of pairs of variables of the ordered bioinformatics data, wherein the exchangeability score is a similarity measure for the variables and the pairs of variables are pairs of variables with an exchangeability score exceeding a predefined threshold value, resulting in an exchangeability matrix $V_\ell$ which carries information about the exchangeability between each of the plurality of measurement variables g such that the entry $(V_\ell)_{ij}$ of the exchangeability matrix $V_\ell$ is the exchangeability score of measurement variables $g_i$ and $g_j$;
expand (130) the ordered list $\ell$ of the data by multiplying the computed exchangeability matrix $V_\ell$ with a computed diagonal matrix $A_\ell$ comprising information of the position of the variables in the ordered list $\ell$ to obtain a representation of the ordered list as an expanded experimental list vector $l_\ell$; and
compare (140) the expanded experimental list vector $l_\ell$ with another list vector comprising a plurality of measurement variables g by computing a dissimilarity score between the expanded experimental list vector $l_\ell$ and the list vector comprising a plurality of measurement variables g resulting in a list of dissimilarity scores, each dissimilarity score corresponding to a specific set of measurement variables g; and to
determine the relationship between the samples by identifying the specific set of measurement variables g having the smallest dissimilarity score and/or the specific set of measurement variables g having the largest dissimilarity score thereby reducing the plurality $i$ into an identified set of data samples being indicative of the biological state.

**Patentansprüche**

1. Computerimplementiertes Verfahren (100) zum Identifizieren einer Menge, welcher eine Beziehung zwischen einer Vielzahl i von Bioinformatikdatenproben von jeder einer Vielzahl von Messvariablen g umfasst, basierend auf einem Rangordnungsverfahren zum Sortieren der Daten, wobei die Beziehung indikativ für einen biologischen Zustand ist, wobei das computerimplementierte Verfahren umfasst:

   Sortieren (110) der Bioinformatikdaten in eine geordnete Liste $\ell$ gemäß der Rangordnung, die durch das Rangordnungsverfahren bereitgestellt wird;
   **dadurch gekennzeichnet, dass** das computerimplementierte Verfahren ferner umfasst:

   Berechnen (120) eines Austauschbarkeit-Scores von Paaren von Variablen der geordneten Bioinformatikdaten, wobei der Austauschbarkeit-Score ein Ähnlichkeitsmaß für die Variablen ist, und wobei die Teilmengen der Variablen Paare von Variablen mit einem Austauschbarkeits-Score sind, der einen vorbestimmten Schwellenwert überschreitet, wodurch eine Austauschbarkeitsmatrix $V_\ell$ resultiert, die Informationen über die Austauschbarkeit zwischen jeder der Vielzahl von Messvariablen g trägt, so dass der Eintrag $(V_\ell)_{ij}$ der Austauschbarkeitsmatrix $V_\ell$ der Austauschbarkeits-Score der Messvariablen $g_i$ und $g_j$ ist;
   Erweitern (130) der geordneten Liste $\ell$ der Daten durch Multiplizieren der berechneten Austauschbarkeitsmatrix $V_\ell$ mit einer berechneten diagonalen Matrix $A_\ell$, die Informationen der Positionen der Variablen in der geordneten Liste $\ell$ umfasst, um eine Repräsentation der geordneten Liste als ein erweiterter experimenteller Listenvektor $I_\ell$ zu erhalten; und
   Vergleichen (140) des erweiterten experimentellen Listenvektors $I_\ell$ mit einem weiteren Listenvektor, der eine Vielzahl von Messvariablen g umfasst, durch Berechnen eines Unterschiedlichkeits-Scores zwischen dem erweiterten experimentellen Listenvektor $I_\ell$ und dem Listenvektor, der eine Vielzahl von Messvariablen g umfasst, wodurch eine Liste von Unterschiedlichkeits-Scores resultiert, wobei jeder Unterschiedlichkeits-Score einer spezifischen Menge von Messvariablen g entspricht; und
   Bestimmen der Beziehung zwischen den Proben durch Identifizieren der spezifischen Menge von Messvariablen g, die den kleinsten Unterschiedlichkeits-Score aufweisen, und/oder der spezifischen Menge von Messvariablen g, die den größten Unterschiedlichkeits-Score aufweisen, wodurch die Vielzahl i in eine identifizierte Menge von Datenproben reduziert wird, die indikativ für den biologischen Zustand ist.

2. Computerimplementiertes Verfahren nach Anspruch 1, wobei das Vergleichen (140) zumindest zweimal mit unterschiedlichen Listenvektoren durchgeführt wird, umfassend eine Vielzahl von Messvariablen g und ein Auswählen eines Listenvektors mit dem niedrigsten und/oder höchsten Unterschiedlichkeits-Scores, wodurch die Vielzahl i in eine identifizierte Menge von Datenproben reduziert wird, die hochgradig indikativ für den biologischen Zustand ist.

3. Computerimplementiertes Verfahren nach Anspruch 1 oder 2, wobei das Erweitern (130) die folgenden Schritte umfasst:

   Berechnen (130a) einer diagonalen Positionsmatrix $A_\ell$, indem diagonale Elemente definiert werden als:

$$(A_\ell)_{ii} = u(r_i)$$

   wobei $r_i$ die Rangordnungsstatistik der Variablen i ist, die beim Sortieren (110) der Daten in eine geordnete Liste $\ell$ verwendet wird, und $u : \rightarrow \rightarrow$ eine monotone Funktion ist;
   Berechnen (130b) einer diagonalen globalen Gewichtungsmatrix $W_\ell$ durch Gewichten der Daten;
   Berechnen (130c) eines erweiterten experimentellen Listenvektors $I_\ell$ gemäß einer Funktion:

$$f(A_\ell, V_\ell, W_\ell) = I_\ell.$$

4. Computerimplementiertes Verfahren nach Anspruch 3, wobei das Berechnen (130c) eines erweiterten experimentellen Listenvektors $I_\ell$ gemäß folgender Formel erfolgt:

$$I_\ell = ((I_\ell)_1, \ldots, (I_\ell)_M)$$

indem gesetzt wird:

$$(I_\ell)_i = h((G_\ell)_i)$$

wobei $G_\ell = A_\ell V_\ell W_\ell$; und $(G_\ell)_i$ die $i$:te Spalte von $G_\ell$ und h: $\mathbb{R}^M \to \mathbb{R}$ ist.

5. Computerimplementiertes Verfahren nach einem der vorangehenden Ansprüche, wobei das Rangordnungsverfahren ist: Signal-zu-Rausch-Verhältnis (SNR), Fold-Change eines durchschnittlichen Expressionswerts, t-Test, ANOVA und nicht-parametrische Tests.

6. Computerimplementiertes Verfahren nach einem der vorhergehenden Ansprüche, wobei das Berechnen (120) eines Austauschbarkeits-Scores jedes Paars von Variablen ist: totaler Austauschbarkeitsvariations-Score, PS, Mittelwertaustauschbarkeits-Score, ES(mean), Maximalaustauschbarkeits-Score, ES(max), einseitiger Mittelwertaustauschbarkeits-Score, oES(mean), einseitiger Maximalaustauschbarkeits-Score, oES(max), oder normalisierte Varianten davon.

7. Verfahren nach einem der vorhergehenden Ansprüche, wobei die Bioinformatikdaten Genexpressionsdaten, microRNA-Daten, DNA-Methylierungsdaten oder Proteinexpressionsdaten sind.

8. Computerprogrammprodukt, umfassend Computerprogrammcodemittel zum Ausführen des Verfahrens nach einem der Ansprüche 1 bis 7, wenn die Computerprogrammcodemittel von einer elektronischen Vorrichtung (200) mit Computerfähigkeiten ausgeführt werden.

9. Computerlesbares Medium (300), auf dem ein Computerprogrammprodukt gespeichert ist, das Computerprogrammcode umfasst, der bewirkt, dass eine elektronische Vorrichtung (200) mit Computerfähigkeiten das Verfahren nach einem der Ansprüche 1 bis 7 ausführt, wenn der Computerprogrammcode in eine Steuerung der elektronischen Vorrichtung (200) geladen und von dieser ausgeführt wird.

10. Vorrichtung zum Identifizieren einer Menge, der eine Beziehung zwischen einer Vielzahl i von Bioinformatikdatenproben von jeder einer Vielzahl von Messvariablen g umfasst, basierend auf einem Rangordnungsverfahren zum Sortieren der Daten, wobei die Beziehung indikativ für einen biologischen Zustand ist, wobei die Vorrichtung einen Speicher zum Speichern von Daten und Anweisungen und eine Steuerung umfasst, wobei die Steuerung zu Folgendem ausgebildet ist:

Sortieren (110) der Bioinformatikdaten in eine geordnete Liste $\ell$ gemäß der Rangordnung, die durch das Rangordnungsverfahren bereitgestellt wird;
**dadurch gekennzeichnet, dass** die Steuerung ferner zu Folgendem ausgebildet ist:

Berechnen (120) eines Austauschbarkeit-Scores von Paaren von Variablen der geordneten Bioinformatikdaten, wobei der Austauschbarkeit-Score ein Ähnlichkeitsmaß für die Variablen ist, und wobei die Paare von Variablen Paare von Variablen mit einem Austauschbarkeits-Score sind, der einen vorbestimmten Schwellenwert überschreitet, wodurch eine Austauschbarkeitsmatrix $V_\ell$ resultiert, die Informationen über die Austauschbarkeit zwischen jeder der Vielzahl von Messvariablen g trägt, so dass der Eintrag $(V_\ell)_{ij}$ der Austauschbarkeitsmatrix $V_\ell$ der Austauschbarkeits-Score der Messvariablen $g_i$ und $g_j$ ist;
Erweitern (130) der geordneten Liste $\ell$ der Daten durch Multiplizieren der berechneten Austauschbarkeitsmatrix $V_\ell$ mit einer berechneten diagonalen Matrix $A_\ell$, die Informationen der Positionen der Variablen in der geordneten Liste $\ell$ umfasst, um eine Repräsentation der geordneten Liste als ein erweiterter experimenteller Listenvektor $I_\ell$ zu erhalten; und
Vergleichen (140) des erweiterten experimentellen Listenvektors $I_\ell$ mit einem weiteren Listenvektor, der eine Vielzahl von Messvariablen g umfasst, durch Berechnen eines Unterschiedlichkeits-Scores zwischen dem erweiterten experimentellen Listenvektor $I_\ell$ und dem Listenvektor, der eine Vielzahl von Messvariablen g umfasst, wodurch eine Liste von Unterschiedlichkeits-Scores resultiert, wobei jeder Unterschiedlichkeits-Score einer spezifischen Menge von Messvariablen g entspricht; und
Bestimmen der Beziehung zwischen den Proben durch Identifizieren der spezifischen Menge von Messvariablen g, die den kleinsten Unterschiedlichkeits-Score aufweisen, und/oder der spezifischen Menge von Messvariablen g, die den größten Unterschiedlichkeits-Score aufweisen, wodurch die Vielzahl i in einer identifizierten Menge von Datenproben reduziert wird, die indikativ für den biologischen Zustand ist.

**Revendications**

1. Procédé mis en œuvre par ordinateur (100) pour identifier un ensemble ayant une relation entre une pluralité i d'échantillons de données bioinformatiques de chacune d'une pluralité de variables de mesure g, sur la base d'un procédé de classement pour trier lesdites données, ladite relation indiquant un état biologique, ledit procédé mis en œuvre par ordinateur comprenant

   le tri (110) des données bioinformatiques en une liste ordonnée $\ell$ conformément au classement fourni par le procédé de classement ;
   **caractérisé en ce que** le procédé mis en œuvre par ordinateur comprend en outre
   le calcul (120) d'un score de possibilité d'échange de paires de variables des données bioinformatiques ordonnées, dans lequel le score de possibilité d'échange est une mesure de similarité pour les variables, et les sous-ensembles de variables sont des paires de variables avec un score de possibilité d'échange dépassant une valeur de seuil prédéfinie, résultant en une matrice de possibilité d'échange $V_\ell$ qui comporte des informations sur la possibilité d'échange entre chacune de la pluralité de variables de mesure g de sorte que l'entrée $(V_\ell)_{ij}$ de la matrice de possibilité d'échange $V_\ell$ soit le score de possibilité d'échange de variables de mesure $g_i$ et $g_j$ ;
   le développement (130) de la liste ordonnée $\ell$ des données en multipliant la matrice de possibilité d'échange $V_\ell$ calculée avec une matrice diagonale $A_\ell$ calculée comprenant des informations de la position des variables dans la liste ordonnée $\ell$ pour obtenir une représentation de la liste ordonnée comme un vecteur liste expérimental développé $l_\ell$ ; et
   la comparaison (140) du vecteur liste expérimental développé $l_\ell$ avec un autre vecteur liste comprenant une pluralité de variables de mesure g en calculant un score de dissimilarité entre le vecteur liste expérimental développé $l_\ell$ et le vecteur liste comprenant une pluralité de variables de mesure g résultant en une liste de scores de dissimilarité, chaque score de dissimilarité correspondant à un ensemble spécifique de variables de mesure g ; et
   la détermination de la relation entre les échantillons en identifiant l'ensemble spécifique de variables de mesure g ayant le plus petit score de dissimilarité et/ou l'ensemble spécifique de variables de mesure g ayant le plus grand score de dissimilarité, réduisant ainsi la pluralité $i$ en un ensemble identifié d'échantillons de données indiquant l'état biologique.

2. Procédé mis en œuvre par ordinateur selon la revendication 1, réalisant en outre la comparaison (140) au moins deux fois avec différents vecteurs listes comprenant une pluralité de variables de mesure g et sélectionnant un vecteur liste avec le score de dissimilarité le plus petit et/ou le plus grand, réduisant ainsi la pluralité $i$ en un ensemble identifié d'échantillons de données indiquant fortement l'état biologique.

3. Procédé mis en œuvre par ordinateur selon la revendication 1 ou 2, dans lequel le développement (130) comprend les étapes
   de calcul (130a) de la matrice de positions diagonale $A_\ell$ en définissant des éléments diagonaux comme :

$$(A_\ell)_{ii} = u(r_i)$$

   où $r_i$ est la statistique de classement de la variable i utilisée dans le tri (110) des données en une liste ordonnée $\ell$,
   et $u : \mathbb{R} \to \mathbb{R}$ est une fonction monotone ;

   le calcul (130b) d'une matrice de poids global diagonale $W_\ell$, en pondérant les données ;
   le calcul (130c) du vecteur liste expérimental développé $l_\ell$ conformément à une fonction :

$$f(A_\ell, V_\ell, W_\ell) = l_\ell.$$

4. Procédé mis en œuvre par ordinateur selon la revendication 3, dans lequel le calcul (130c) d'un vecteur liste expérimental développé $l_\ell$ est fait conformément à la formule :

$$l_\ell = ((l_\ell)_1, \ldots, (l_\ell)_M)$$

en laissant

$$(l_\ell)_i = h((G_\ell)_i)$$

où $G_\ell = A_\ell V_\ell W_\ell$ ; et $(G_\ell)_i$ est la i:ème colonne de $G_\ell$ et $h : \mathbb{R}^M \to \mathbb{R}$ .

5. Procédé mis en œuvre par ordinateur selon l'une quelconque des revendications précédentes, dans lequel le procédé de classement est un rapport signal sur bruit (SNR), un facteur de régulation de valeur d'expression moyenne, un test t, une ANOVA et des tests non paramétriques.

6. Procédé mis en œuvre par ordinateur selon l'une quelconque des revendications précédentes, dans lequel le calcul (120) d'un score de possibilité d'échange de chaque paire de variables est un score de variation de possibilité d'échange total, PS, un score de possibilité d'échange moyen, ES(mean), un score de possibilité d'échange maximal, ES(max), un score de possibilité d'échange moyen unilatéral, oES(mean), un score de possibilité d'échange maximal unilatéral, oES(max) ou des variantes normalisées de ceux-ci.

7. Procédé selon l'une quelconque des revendications précédentes, dans lequel les données bioinformatiques sont des données d'expression génique, des données de microARN, des données de méthylation de l'ADN ou des données d'expression de protéines.

8. Produit programme d'ordinateur comprenant des moyens de code de programme d'ordinateur pour exécuter le procédé selon l'une quelconque des revendications 1 à 7 lorsque lesdits moyens de code de programme d'ordinateur tournent sur un dispositif électronique (200) ayant des capacités informatiques.

9. Support lisible par ordinateur (300) ayant stocké sur celui-ci un produit programme d'ordinateur comprenant un code de programme d'ordinateur qui amène un dispositif électronique (200) ayant des capacités informatiques à exécuter le procédé selon l'une quelconque des revendications 1 à 7 lorsque ledit code de programme d'ordinateur est chargé dans et exécuté par un dispositif de commande du dispositif électronique (200) .

10. Appareil pour identifier un ensemble ayant une relation entre une pluralité *i* d'échantillons de données bioinformatiques de chacune d'une pluralité de variables de mesure g, sur la base d'un procédé de classement pour trier lesdites données, ladite relation indiquant un état biologique, ledit appareil comprenant une mémoire pour stocker des données et des instructions et un dispositif de commande, dans lequel ledit dispositif de commande est configuré pour

trier (110) les données bioinformatiques en une liste ordonnée $\ell$ conformément au classement fourni par le procédé de classement ;
**caractérisé en ce que** le dispositif de commande est en outre configuré pour
calculer (120) un score de possibilité d'échange de paires de variables des données bioinformatiques ordonnées, dans lequel le score de possibilité d'échange est une mesure de similarité pour les variables et les paires de variables sont des paires de variables avec un score de possibilité d'échange dépassant une valeur de seuil prédéfinie, résultant en une matrice de possibilité d'échange $V_\ell$ qui comporte des informations sur la possibilité d'échange entre chacune de la pluralité de variables de mesure g de sorte que l'entrée $(V_\ell)_{ij}$ de la matrice de possibilité d'échange $V_\ell$ soit le score de possibilité d'échange de variables de mesure $g_i$ et $g_j$ ;
développer (130) la liste ordonnée $\ell$ des données en multipliant la matrice de possibilité d'échange $V_\ell$ calculée avec une matrice diagonale $A_\ell$ calculée comprenant des informations de la position des variables dans la liste ordonnée $\ell$ pour obtenir une représentation de la liste ordonnée comme un vecteur liste expérimental développé $l_\ell$ ; et
comparer (140) le vecteur liste expérimental développé $l_\ell$ avec un autre vecteur liste comprenant une pluralité de variables de mesure g en calculant un score de dissimilarité entre le vecteur liste expérimental développé $l_\ell$ et le vecteur liste comprenant une pluralité de variables de mesure g résultant en une liste de scores de dissimilarité, chaque score de dissimilarité correspondant à un ensemble spécifique de variables de mesure g ; et pour

déterminer la relation entre les échantillons en identifiant l'ensemble spécifique de variables de mesure g ayant le plus petit score de dissimilarité et/ou l'ensemble spécifique de variables de mesure g ayant le plus grand score de dissimilarité, réduisant ainsi la pluralité *i* en un ensemble identifié d'échantillons de données indiquant l'état biologique.

Fig. 1

Fig. 2A

Fig. 2B

## Overlap Extended Top

| 10 | 9 | 8 | 9 | 8 | 9 | 8 | 9 | 9 | 8 |
|----|----|----|----|----|----|----|----|----|----|
| 9 | 10 | 8 | 9 | 9 | 9 | 9 | 8 | 8 | 9 |
| 8 | 8 | 10 | 9 | 9 | 8 | 9 | 8 | 7 | 7 |
| 9 | 9 | 9 | 10 | 9 | 9 | 9 | 9 | 8 | 8 |
| 8 | 9 | 9 | 9 | 10 | 8 | 10 | 8 | 7 | 8 |
| 9 | 9 | 8 | 9 | 8 | 10 | 8 | 8 | 8 | 9 |
| 8 | 9 | 9 | 9 | 10 | 8 | 10 | 8 | 7 | 8 |
| 9 | 8 | 8 | 9 | 8 | 8 | 8 | 10 | 9 | 8 |
| 9 | 8 | 7 | 8 | 7 | 8 | 7 | 9 | 10 | 8 |
| 8 | 9 | 7 | 8 | 8 | 9 | 8 | 8 | 8 | 10 |

Fig. 3A

## Overlap Extended Bottom

| 10 | 6 | 5 | 5 | 6 | 9 | 6 | 8 | 8 | 8 |
|----|----|----|----|----|----|----|----|----|----|
| 6 | 10 | 6 | 7 | 6 | 6 | 7 | 5 | 6 | 6 |
| 5 | 6 | 10 | 7 | 7 | 6 | 7 | 4 | 6 | 6 |
| 5 | 7 | 7 | 10 | 6 | 6 | 5 | 4 | 6 | 6 |
| 6 | 6 | 7 | 6 | 10 | 6 | 8 | 5 | 7 | 8 |
| 9 | 6 | 6 | 6 | 6 | 10 | 5 | 8 | 8 | 8 |
| 6 | 7 | 7 | 5 | 8 | 5 | 10 | 4 | 6 | 6 |
| 8 | 5 | 4 | 4 | 5 | 8 | 4 | 10 | 8 | 7 |
| 8 | 6 | 6 | 6 | 7 | 8 | 6 | 8 | 10 | 8 |
| 8 | 6 | 6 | 6 | 8 | 8 | 6 | 7 | 8 | 10 |

Fig. 3B

EP 2 684 150 B1

Overlap Not extended Top

Fig. 4A

Overlap Not extended Bottom

Fig. 4B

## Overlap GSEA Top

| 10 | 3 | 0 | 0 | 0 | 2 | 1 | 1 | 4 | 1 |
|----|----|----|----|----|----|----|----|----|----|
| 3 | 10 | 3 | 3 | 3 | 0 | 0 | 2 | 2 | 2 |
| 0 | 3 | 10 | 1 | 2 | 0 | 1 | 2 | 2 | 2 |
| 0 | 3 | 1 | 10 | 3 | 0 | 2 | 4 | 0 | 1 |
| 0 | 3 | 2 | 3 | 10 | 0 | 0 | 1 | 1 | 2 |
| 2 | 0 | 0 | 0 | 0 | 10 | 0 | 1 | 3 | 2 |
| 1 | 0 | 1 | 2 | 0 | 0 | 10 | 4 | 1 | 1 |
| 1 | 2 | 2 | 4 | 1 | 1 | 4 | 10 | 1 | 3 |
| 4 | 2 | 2 | 0 | 1 | 3 | 1 | 1 | 10 | 3 |
| 1 | 2 | 2 | 1 | 2 | 2 | 1 | 1 | 3 | 10 |

Fig. 5A

## Overlap GSEA Bottom

| 10 | 2 | 0 | 2 | 3 | 3 | 3 | 5 | 4 | 4 |
|----|----|----|----|----|----|----|----|----|----|
| 2 | 10 | 0 | 2 | 2 | 1 | 1 | 2 | 3 | 3 |
| 0 | 0 | 10 | 0 | 2 | 0 | 0 | 0 | 1 | 1 |
| 2 | 2 | 0 | 10 | 1 | 2 | 3 | 2 | 1 | 3 |
| 3 | 2 | 2 | 1 | 10 | 0 | 1 | 2 | 2 | 3 |
| 3 | 1 | 0 | 2 | 0 | 10 | 2 | 3 | 3 | 2 |
| 3 | 1 | 0 | 3 | 1 | 2 | 10 | 4 | 1 | 5 |
| 5 | 2 | 0 | 2 | 2 | 3 | 4 | 10 | 1 | 5 |
| 4 | 3 | 1 | 1 | 2 | 3 | 1 | 1 | 10 | 3 |
| 4 | 3 | 1 | 3 | 3 | 2 | 5 | 5 | 3 | 10 |

Fig. 5B

**Gene sets related to poor outcome**

| The invention | | GSEA | |
|---|---|---|---|
| Gene set | Distance | Gene set | NES |
| LEU_DOWN | 1.082 | p53hypoxiaPathway | -2.0334 |
| GLUT_DOWN | 1.075 | MAP00970_Aminoacyl_tRNA_biosynthesis | -1.8772 |
| INSULIN_2F_UP | 1.071 | INSULIN_2F_UP | -1.8554 |
| proteasomePathway | 1.063 | tRNA_Synthetases | -1.7979 |
| MAP00970_Aminoacyl_tRNA_biosynthesis | 1.053 | LEU_DOWN | -1.7816 |
| Proteasome_Degradation | 1.051 | HTERT_UP | -1.7733 |
| tRNA_Synthetases | 1.051 | GLUT_DOWN | -1.7415 |
| p53hypoxiaPathway | 1.048 | cell_cycle_checkpoint | -1.6716 |
| RAP_DOWN | 1.048 | proteasomePathway | -1.6287 |
| HTERT_UP | 1.041 | Proteasome_Degradation | -1.5639 |

**Gene sets related to good outcome**

| The invention | | GSEA | |
|---|---|---|---|
| Gene set | Distance | Gene set | NES |
| GPCRs_Class_A_Rhodopsin-like | 0.937 | ST_Ga12_Pathway | 1.8126 |
| mitochondr | 0.947 | SIG_BCR_Signaling_Pathway | 1.6675 |
| ST_Ga12_Pathway | 0.948 | ANTI_CD44_UP | 1.6396 |
| MAP00650_Butanoate_metabolism | 0.951 | SIG_PIP3_signaling_in_B_lymphocytes | 1.6035 |
| ANTI_CD44_UP | 0.953 | MAP00590_Prostaglandin_and_leukotriene_metabolism | 1.5294 |
| human_mitoDB_6_2002 | 0.953 | tnf_and_fas_network | 1.5136 |
| SIG_BCR_Signaling_Pathway | 0.953 | MAP00561_Glycerolipid_metabolism | 1.5132 |
| MAP00071_Fatty_acid_metabolism | 0.956 | MAP00071_Fatty_acid_metabolism | 1.4766 |
| PROLIF_GENES | 0.957 | cell_cycle_regulator | 1.4742 |
| fcer1Pathway | 0.961 | Fatty_Acid_Degradation | 1.4672 |

Fig. 6

Fig. 7

Fig. 8

300

200

## Fig. 9

(1.46e-3, -1.41e-4, -1.32e-1, -1.02e-3, 2.17e-3, 9.98e-4, 1.24e-1, 4.96e-1, -6.62e-4, 4.19e-1, ...)

## Fig. 13

Fig. 10

$$
\begin{pmatrix}
1 & 0.22 & 0 & 0 & 0 & 0.066 & 0.16 & 0 & 0 & 0 \\
0.22 & 1 & 0 & 0 & 0.018 & 0.43 & 0 & 0 & 0.13 & 0 \\
0 & 0 & 1 & 0 & 0 & 0 & 0 & 0 & 0.086 & 0 \\
0 & 0 & 0 & 1 & 0 & 0 & 0 & 0 & 0.079 & 0 \\
0 & 0.018 & 0 & 0 & 1 & 0 & 0.30 & 0 & 0 & 0.11 \\
0.066 & 0.43 & 0 & 0 & 0 & 1 & 0.41 & 0 & 0 & 0 \\
0.16 & 0 & 0 & 0 & 0.30 & 0.41 & 1 & 0.32 & 0 & 0.24 \\
0 & 0 & 0 & 0 & 0 & 0 & 0.32 & 1 & 0 & 0.61 \\
0 & 0.13 & 0.086 & 0.079 & 0 & 0 & 0 & 0 & 1 & 0 \\
0 & 0 & 0 & 0 & 0.11 & 0 & 0.24 & 0.61 & 0 & 1
\end{pmatrix}
$$

Fig. 11

Fig. 12

**EP 2 684 150 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JURMAN et al.** *Algebraic Comparison of Partial Lists in Bioinformatics,* 08 April 2010, https://arxiv.org/pdf/1004.1341.pdf